# EUROPÄISCHE PATENTSCHRIFT

(11) **EP 3 875 145 B1**
(45) Veröffentlichungstag und Bekanntmachung des Hinweises auf die Patenterteilung: **16.02.2022**
(21) Anmeldenummer: 20171941.6
(22) Anmeldetag: 28.04.2020
(51) Int. Cl.: A61N 5/06

(54) **LÄNGLICHES BELEUCHTUNGSMITTEL MIT WENIGSTENS EINER IM BLAULICHTBEREICH LICHT ABGEBENDEN LICHTQUELLE**
ELONGATED LIGHTING MEANS WITH AT LEAST ONE LIGHT SOURCE EMITTING LIGHT IN THE BLUE RANGE
MOYEN D'ÉCLAIRAGE OBLONG AVEC AU MOINS UNE SOURCE LUMINEUSE ÉMETTANT DE LA LUMIÈRE DANS LE DOMAINE BLEU

(30) Priorität: 02.03.2020 DE 102020105567; 03.04.2020 DE 202020101826 U
(43) Veröffentlichungstag der Anmeldung: 08.09.2021
(73) Patentinhaber: RP-Technik GmbH, 63110 Rodgau (DE)
(72) Erfinder: PASEDAG, Reinald, 63110 Rodgau (DE); PASEDAG, Roland, 63110 Rodgau (DE)
(74) Vertreter: Cremer & Cremer

(56) Entgegenhaltungen:
- WO-A1-2011/083378
- DE-A1-102012 109 602
- KR-B1- 101 858 657
- KR-B1- 102 025 614
- US-A1- 2003 009 205
- US-A1- 2004 193 235
- US-A1- 2010 076 526

## Beschreibung

Die vorliegende Erfindung behandelt ein Mundsteckstück, das teilweise in den Mund eines Lebewesens wie eines Menschen eingeführt werden kann, um insbesondere den Gesundheitszustand des Menschen zu fördern oder zu verbessern.

Mit anderen Worten, die vorliegende Erfindung behandelt ein Mundsteckstück nach dem Oberbegriff von Anspruch 1.

### Technisches Gebiet

Aus zahlreichen medizinischen Untersuchungen ist bekannt, dass mit Hilfe von Licht ausgewählter Wellenlängen positive Effekte bei äußerlich bestrahlten Personen sowie bei einer Effusion von Körperflüssigkeiten wie Blut oder Plasma (siehe z. B. "A new proof of concept in bacterial reduction" von Michelle Maclean et al. in Journal of Blood Transfusion, Volume 2016) herbeigeführt werden kann. Sowohl aus der Fachliteratur als auch aus der Patentliteratur lassen sich Hinweise entnehmen, dass nicht nur ultraviolettes Licht und infrarotes Licht positive Effekte bei dem Bestrahlen von Lebewesen, wie Menschen oder Tieren, sondern auch einzelne Frequenzen und Frequenzbänder im optischen bzw. sichtbaren Wellenspektrum ebenfalls positive Effekte hervorrufen können.

Das für Menschen sichtbare Wellenspektrum wird üblicherweise in einem Wellenlängenbereich zwischen 400 nm und einem Wellenlängenbereich unterhalb von 750 nm angesiedelt. Die Breite und die exakte Lage des Frequenzbandes, also welche Wellenlängen tatsächlich sichtbarem Licht entsprechen, bzw. die Wellenlängengrenzwerte des Wellenlängenbandes variieren etwas von Mensch zu Mensch. Auch gilt bei manchen Tieren ein etwas anderes Wellenband als sichtbares Lichtwellenband, d. h. in dem jene Tiere Licht optisch wahrnehmen können (z. B. ist das Wellenlängenband bei Honigbienen um ca. 150 nm in Richtung auf das kurzwelligere UV-Licht verschoben). Generell kann aber gesagt werden, dass als optisch sichtbares Wellenlängenband ein Wellenlängenband angesehen werden kann, das zwar in den Randbereichen bzw. bezüglich seiner Grenzwerte um einige nm variieren kann, das jedoch eine Bandbreite von ca. 350 nm überstreicht. Dieser Wellenlängenbereich wird gemeinhin als sichtbarer Wellenlängenbereich bezeichnet, dessen Grenzwerte auf das jeweils adressierte Lebewesen konkret anzupassen sind.

Außerhalb des sichtbaren Wellenlängenbereichs ist der ultraviolette Wellenlängenbereich angesiedelt. Ultraviolettes Licht in vernünftigen Dosen hat medizinisch positive Wirkung. Es ist aber auch aus der Fachliteratur genauso wie aus der Patentliteratur bekannt, dass der Sonnenbestrahlung der Effekt der aktinischen Keratose zugeordnet wird.

Auch lassen sich in der Fachliteratur Stimmen finden, die erläutern, dass gewisse Wellenlängen im blauen Spektrum und/oder violetten Spektrum verschiedene positive Effekte beim Menschen hervorrufen können sollen.

So erläutert z. B. der Fachaufsatz "New Proof-of-Concept in Viral Inactivation: Virucidal Efficacy of 405 nm Light Against Feline Calicivirus as a Model for Norovirus Decontamination" von Rachael M. Tomb, Michelle Maclean, John E. Coia, Elizabeth Graham, Michael McDonald, Chintamani D. Atreya, Scott J. MacGregor und John G. Anderson, veröffentlicht am 31. Dezember 2016 in "Food and Environmental Virology", dass mit Hilfe eines Lichts der Wellenlänge 405 nm bakterizide, fungizide und auch viruzide Inaktivierungen durch oxidative Schädigung der Bakterien, Pilze und Viren herbeigeführt werden kann.

Der Fachaufsatz von der University of Arizona mit dem Titel "Blue light can help heal mild traumatic brain injury", veröffentlicht am 15. Januar 2020 auf Science Daily, schlägt vor, morgens eine Blaulichttherapie Leuten zuteilwerden zu lassen, die ein Gehirntrauma erlitten haben.

Aus dem Fachaufsatz "The Influence of Simulated Sunlight on the Inactivation of Influenza Virus in Aerosols" der Autoren Michael Schuit et al., veröffentlicht am 28. November 2019 durch Journal of Infectious Diseases (JID 2020:221), kann die Schlussfolgerung abgeleitet werden, dass die Übertragung von Grippe-Viren im Freien unter Tageslicht höchstunwahrscheinlich ist, aber eine Übertragung bei schlechteren UV-Lichtverhältnissen und über kurze Distanzen gegeben ist.

Der am 01. Mai 2020 nachveröffentlichte Artikel "Light as a potential treatment for pandemic coronavirus infections: A perspective" der Autoren Chukuka Samuel Enwemeka et al. aus Journal of Photochemistry & Photobiology ist eine Literaturarbeit, die die Behauptung aufstellt, dass eine im Bereich eines violett-blauen Lichts angesiedelte Bestrahlung Auswirkungen auf Bakterien habe, sodass hierdurch Tuberkulose behandelbar sei.

Aus verschiedenen Quellen lässt sich folglich ableiten, dass Blaulicht dem Gesundheitszustand von Menschen zuträglich ist, nicht zuletzt, wenn humoristische oder karnevalistische Aspekte hinzukommen.

### Stand der Technik

Gegenstände mit Leuchtmitteln, die speziell für Lichtabgabe im Nahbereich des Mundes eines Menschen vorgesehen sind, lassen sich z. B. der EP 1 273 208 B1 (Inhaber: Mermaz, Christophe et al.; Erteilungstag: 14.01.2004) entnehmen, die einen leuchtenden Lutscher vorstellt. Als ein Ausführungsbeispiel ist vorgesehen, einem Lutscher eine gefärbte Zuckerhülle zu geben, welche transparent oder transluzent ein Leuchtmodul umgibt.

Aus Zahnarztpraxen und aus der DE 10 2018 119 423 A1 (Anmelderin: Schott AG; Offenlegungstag: 13.02.2020) sind Lichthärtegeräte für Zahnplombierungsmaterial (den so genannten "composites") bekannt, die Licht auf Zähne mit Kunststofffüllungen aussenden sollen, um eine Kunststofffüllung (bzw. ein "composite") in einem reparierten Zahn zu härten ("Lichtpolymerisationsgeräte"). Hierbei schlägt die DE 10 2018 119 423 A1 vor, blaues Licht mit der Wellenlänge von 405 nm zu verwenden, um z. B. im Bereich der Backenzähne eine Aushärtung flüssiger Materialien, z. B. von Klebern, durchzuführen.

Die DE 25 35 200 A (Anmelderin: CAVITRON CORP; Offenlegungstag: 02.12.1976) behauptet, die auf der Welt in Gestalt eines Schutzrechts erschienene zweite Beschreibung eines UV-Bestrahlungsgeräts für die Zahnheilkunde zum Aushärten eines Harzes zu sein (siehe S. 6, Mitte der DE 25 35 200 A), wobei die zu verwendende UV-Lampe eine Hochspannungsgasentladungslampe ist. In einer Ausführungsform wird eine Lampenform vorgestellt, die durch ihre Rundung der Zahnreihung und dem Bogen von Zähnen im Kiefer folgend nachempfunden ist. Die Beschreibung der DE 25 35 200 A hebt hervor, dass ein möglichst dünnes Kalkglasröhrchen, das nur etwa 6,4 mm im Durchmesser betragen soll, zu verwenden ist, damit ein Zahnarzt seinem Patienten das UV-Bestrahlungsgerät so einführen kann, dass ein in einem Zahn eingebrachter polymerer Kunstharz im Wege der Bestrahlung aushärten kann.

Ein weiteres Gerät für die Photopolymerisation von Zahnreparaturmaterial wie einem aushärtbaren Harz wird in der Figur 2 der US 2017/035 539 A1 (Anmelder: Joseph F. Bringley; Veröffentlichungstag: 09. Februar 2017) vorgestellt. Eine lichtleitende Spitze ist gebogen ausgeführt, damit das Licht zielgerichteter auf einen Zahn mit einer organischen Kunststoffmatrix als Füllung zu ihrer Härtung aufgebracht werden kann.

Für eine orale Anwendung wird in der WO 2018/213 893 A1 (Anmelderin: Cosmoaesthetics Pty Ltd; Veröffentlichungstag: 29.11.2018) ein mit LEDs ausgestatteter Griff beschrieben, an den ein austauschbares Mundstück angesteckt werden kann, nachdem durch einen Schnell-Auslöse-Mechanismus das nicht mehr benötigte Mundstück von dem Griff getrennt worden ist.

Ein weiteres Gerät für eine Anwendung im Mundbereich einer Person wird in der US 2008/065 175 A1 (Erfinder: Russell J. Redmond et al.; Veröffentlichungstag: 13.03.2008) beschrieben, das eine UV-Lichtquelle für die Abgabe von UV-Strahlen hat.

Weitere stabartige Geräte, die entweder für den Einsatz in einem Mund eines Menschen bestimmt sind oder hierfür verwendet werden können, sind der US 2015/030 989 A1 (Anmelder: Nikolaos S. Soukos et al.; Veröffentlichungstag: 29.01.2015), der US 2007/073 362 A1 (Erfinder: Noel Rodney Campbell; Veröffentlichungstag: 29.03.2007), der US 6 159 236 A

(Anmelderin: Advanced Photodynamic Technologies, Inc.; Veröffentlichungstag: 12.12.2000), der US 2010/076 526 A1 (Erfinder: Yosef Krespi et al.; Veröffentlichungstag: 25.03.2010) und der US 2010/274 328 A1 (Erfinder: Robert Morgan; Veröffentlichungstag: 28.10.2010) zu entnehmen.

Alle diese Geräte scheinen für eine Nutzung durch fachkundiges Personal wie z. B. einen Zahnarzt gestaltet zu sein. Bei einigen Geräten soll ein Bediener das Gerät an dem Griff während seines Einsatzes im Mund einer Person fortwährend festhalten und so an die richtige Stelle, zum Beispiel in den Bereich einer zu schließenden Zahnkavität, führen.

Weitere medizinische Behandlungsgeräte werden in der WO 2011/083 378 A1 (Anmelderin: Konin-Klijke Philips Electronics N.V.; Veröffentlichungstag: 14. Juli 2011), in der US 2004/193 235 A1 (Erfinder: Altshuler et al.; Veröffentlichungstag: 30. September 2004), in der US 2010/076 526 A1 (Erfinder: Krespi et al.; Veröffentlichungstag: 25. März 2010) und in der DE 10 2012 109 602 A1 (Anmelderin: Pierenkemper GmbH; Offenlegungstag: 14. August 2013) vorgestellt, die unterschiedliche Mundsteckstücke darstellen, mit denen medizinische oder sogar therapeutische Effekte im Mundraum eines Nutzers bewirkbar sein sollen. Die einzelnen Mundsteckstücke unterscheiden sich unter einander vor allem durch ihre Formgebungen, aber auch durch das adressierte Anwendungsgebiet, wie z. B. Tumorbehandlungen oder als Beatmungshilfsgerät.

Gänzlich anders geformte, eher mit Zahnspangen oder auch mit zahnärztlichen Abformlöffeln vergleichbare Mundstücke sind der KR 102 025 614 B1 (Inhaberin: Charmsaramdental Co. Ltd.; Offenlegungstag: 2. September 2019), der US 2003/009 205 A1 (Erfinder: Merrill A. Biel, Veröffentlichungstag: 9. Januar 2003) und der KR 101 858 657 B1 (Inhaberin: Univ Dankook Cheonan Campus Ind Academic Cooperation Foundation; Offenlegungstag: 10. Mai 2018) zu entnehmen. Außerdem stellt die US 2003/009 205 A1 (Erfinder: Merrill A. Biel, Veröffentlichungstag: 9. Januar 2003)ein flächiges photodynamisches Therapiegerät vor.

Werden die zuletzt genannten Druckschriften miteinander verglichen, sind die unterschiedlichen Ausführungsbeispiele besonders variantenreich, die in der US 2004/193 235 A1 vorgestellt werden. Die meisten in der US 2004/193 235 A1 dargestellten Mundsteckstücke ähneln sehr stark klassischen Zahnbürsten. Die eine oder andere Darstellung weicht aber von dem Hauptthema "Licht-Zahnbürste", dem sich die US 2004/193 235 A1 widmet, aufgrund ihrer Gestalt und Formgebung ab, sodass durch punktuelle Entnahme einzelner Merkmale aus verschiedenen Ausführungsbeispielen zahlreiche, interessante Kombinationen zu einzelnen Aspekten möglicher Mundsteckstücke zusammenstellbar sind. Bei einer derart flexiblen Ableitung einzelner Aspekte aus den verschiedenen Ausführungsbeispielen der US 2004/193 235 A1 mag das Dokument als nächst kommender Stand der Technik für die nachfolgend dargestellte Erfindung anzusehen sein.

Aus einem anderen Blickwinkel könnte jedoch auch die WO 2011/083 378 A1 als nächst kommender Stand der Technik betrachtet werden, weil sie eine Saugvorrichtung beschreibt, durch die durch UV-C-Wellen eine Bakterienreduktion und eine Desinfektion im Anwendungsgebiet "Mund" bewirkt werden könnte.

### Aufgabenstellung

Wissenschaftlicher Kenntnisstand zum Anmeldezeitpunkt:
Leute, die Sorge vor Infektionskrankheiten wie einem grippalen Effekt haben, nutzen häufig gerne medizinische Tüchlein oder Mundschutze, die sie sich vor den Mund binden, weil sie glauben, diese Maßnahme stelle eine wirksame Barriere für Viren und Bakterien aus der Umwelt dar und aufgrund des Tüchleins sei ein wirksamer Schutz für ihre Atemwege sichergestellt. In der Fachliteratur gibt es jedoch zahlreiche renommierte Stimmen, die die medizinische Wirkung in Abrede stellen und behaupten, solche Mundschutze stellten nur ein psychologisches Massenphänomen dar.

Tatsächlich ist es wünschenswert, Maßnahmen zu kennen, wie Infekte, Ansteckungen und vor allem über Atemwege übertragene Krankheiten in ihren Wirkungen und Schädigungen verringert werden können. Mit anderen Worten, ein geeigneter Ersatz für solche, wenigstens teilweise heftig kritisierten Schutzmaßnahmen wie ein Mundschutz sollte durch effektivere Maßnahmen und Geräte ermöglicht werden können.

Wissenschaftlicher Kenntnisstand zum Erteilungszeitpunkt:
Zwischenzeitlich sehen Gesundheitsinstitutionen wie das Robert-Koch-Institut es als erwiesen an, das medizinische Masken ein Infektionsgeschehen im zweistelligen Prozentbereich reduzieren können. Selbst hergestellte Tüchlein sollen aber nur deutlich schlechter vor Infektionskrankheiten schützen.

In diesem Zusammenhang wäre es besonders günstig, einen Entwurf für ein Gerät zu kennen, der aufgrund seiner Gestaltung besonders leicht zu bedienen ist, idealerweise auch von weniger geübten Personen.

### Erfindungsbeschreibung

Die erfindungsgemäße Aufgabe wird durch ein Mundsteckstück nach Anspruch 1 gelöst. Vorteilhafte Weiterbildungen lassen sich den abhängigen Ansprüchen entnehmen.

Ein Mundsteckstück ist ein Gegenstand, der dazu vorgesehen ist, in den Mund einer Person oder in das Maul (bzw. Mund) eines Tieres gesteckt zu werden, wobei es erwünscht ist, dass das Mundsteckstück nicht in Gänze im das Mundsteckstück aufnehmenden Menschen oder Tier verschwindet.

Für den Teil, der im Mund verschwinden soll, hat das Mundsteckstück einen Mundhöhlenkörper.

Neben dem Mundhöhlenkörper hat das Mundsteckstück eine Anlagefläche, die auch als Schild zu bezeichnen ist.

Das Mundsteckstück insgesamt bzw. globaler betrachtet kann eine beliebige Formgebung haben, z. B. abschnittsweise oval oder sphärisch geformt sein. Das Mundsteckstück hat aber insgesamt eine längliche Erstreckung. Die längliche Erstreckung kann mit einer Längserstreckung angegeben werden. Hierzu ist die Anlagefläche im (nahezu) 90-Grad-Winkel angeordnet. In diesem Fall verläuft die Anlagefläche quer zur Längserstreckung.

Der Mundhöhlenkörper hat einen Körper, der zumindest in einem Bereich transluzent ausgestaltet ist. Der transluzente Körper hat einen Abschnitt, durch den Licht durchtreten kann. Natürlich kann der gesamte Körper als transluzenter Körper realisiert sein. Besonders vorteilhaft ist es, wenn zumindest der vordere Bereich des stabartigen Mundsteckstücks transluzent ist, z. B. aus einem transluzenten Silikon hergestellt ist.

Im Inneren des Mundsteckstücks ist eine Lichtquelle angeordnet. Die Lichtquelle ist für die Abgabe von Licht bestimmter Wellenlängen bzw. für Wellenlängenbereiche konzipiert.

Das von der Lichtquelle abgegebene Licht liegt vorteilhafterweise in einem Wellenlängenbereich, in dem krankheitsverursachende Fremdkörper wie Bakterien, Viren und/oder Pilze geschädigt, abgetötet oder zumindest in ihrer Keimzahl verringert werden können.

Der Mundhöhlenkörper ist besonders geformt. Hierdurch ist es möglich, dass der. Mundhöhlenkörper eine dentogingivale Anlagerung im Mund herstellen kann. Darüber hinaus ist der Mundhöhlenkörper in einem weiteren Bereich seines Körpers so gestaltet, dass eine linguale Aufnahme des Mundhöhlenkörpers, insbesondere durch einen anderen Bereich des Mundhöhlenkörpers, realisierbar ist. Mit anderen Worten, der Mundhöhlenkörper soll oral zwischen der Zunge und dem Gaumen, hinter den Zähnen aufgenommen werden.

Der Mundhöhlenkörper hat vorteilhafterweise eine längliche Form. Wird der Mundhöhlenkörper schlanker gestaltet, so ist er eher durch den Begriff "stabartig" zu beschreiben. Auch eine Zapfenform ist eine geeignete Form für den Mundhöhlenkörper. Ist der Mundhöhlenkörper nicht nur gerade gestaltet, sondern leicht geschwungen bzw. gebogen ausgestaltet, so kann der vordere Teil des Mundsteckstücks der Gaumenwölbung und/oder der Zungenwölbung nachgeführt sein, allgemeiner ausgedrückt, der Mundwölbung, hervorgerufen durch Gaumen und/oder Zunge, nachempfunden. In einer solchen Gestaltung kann das eine Ende des Mundhöhlenkörpers niedriger liegen als ein mittlerer Bereich des Mundhöhlenkörpers. Das andere Ende des Mundhöhlenkörpers kann ebenfalls niedriger liegen. Beide Enden des Mundhöhlenkörpers können auf ungefähr der gleichen Höhe angesiedelt sein. In einem mittleren Bereich des, insbesondere stabartigen, Mundhöhlenkörpers ist dieser, z. B. im Vergleich zu den Rand- oder Endbereichen des Mundhöhlenkörpers, angehoben.

In einer weiteren alternativen Ausführungsform ist das Mundsteckstück zapfenartig ausgestaltet. Auch kann das schnullerartige Mundsteckstück zapfenartig gestaltet sein.

Die Anlagefläche erstreckt sich in eine andere Richtung als der Mundhöhlenkörper sich erstreckt. Die Anlagefläche erstreckt sich quer zu einer Längserstreckung des Mundsteckstücks.

Als ein Mundschutz zwischen dem Mundhöhlenkörper und einem kastenartigen Körper ist die Anlagefläche zylindermantelartig gewölbt. Die Anlagefläche ist konkav dem Mundhöhlenkörper zugewandt.

Die Anlagefläche ist als Anschlag, Bremse bzw. Stoppelement ausgestaltet, damit ein Teil des Mundsteckstücks außerhalb des Mundes verbleibt. Da die Anlagefläche eine Wölbung bzw. einen Bogen (einen Kreisbogenausschnitt) zeigt, kann die Anlagefläche besonders vorteilhaft einen Mund eines Menschen, insbesondere vollflächig, abdecken und zugleich den Eintritt von Fremdkörpern und Fremdpartikeln, wie Viren oder Bakterien, reduzieren oder sogar verhindern.

Die Anlagefläche ist zum Aufliegen auf einem Teil des Gesichts gestaltet (bzw. der Schnauze, z. B. eines Hundes). Sie liegt beim Menschen zumindest auf dem Amorbogen an. Typischerweise wird die Anlagefläche jedoch auf Teilbereichen sowohl der Oberlippe als auch der Unterlippe anliegen. Hierdurch wird die Auflage bzw. Anlagefläche verteilt, wenn das Mundsteckstück im Mund eingesetzt ist.

Erfindungsgemäß liegen die Wellenlängen im Blaulichtbereich (mittlerer und oberer 400 nm-Bereich), ggf. zusätzlich im Violettlichtbereich (unterer 400 nm-Bereich) und/oder im Ultraviolettlichtbereich (unterhalb von 400 nm, in einem Bereich von bis zu 100 nm).

Mit einem solchen Mundsteckstück ist es möglich, die Keimzahl im Mundbereich oder auch im Rachenbereich eines das Mundsteckstück aufnehmenden Lebewesens, z. B. eines Menschen, zu reduzieren. Hierzu wird das Mundsteckstück in die Mundhöhle des Lebewesens eingeführt. Anschließend wird das Mundsteckstück eingeschaltet, sodass es Licht, insbesondere über seinen transluzenten Bereich des Mundhöhlenkörpers, abgeben kann.

Mit anderen Worten, das Mundsteckstück kann als keimtötendes Werkzeug, insbesondere in einem Mund eines Nutzers, verwendet werden. Ein solches Gerät kann als Desinfektionsgerät bezeichnet werden.

Aus einem vorderen Bereich des Mundsteckstücks, d. h. aus einem Bereich des Mundhöhlenkörpers, kann das Licht austreten.

Das Licht soll auf den Rachenbereich, insbesondere einen Mundschleimhautbereich und vorzugsweise einen Zungenbereich, der Mundhöhle einstrahlen. Es ist besonders vorteilhaft, wenn das Licht gerichtet in den Posteriori-Bereich der Mundhöhle strahlt.

Dazu kann das zuvor vorgestellte Mundsteckstück genutzt werden.

Durch ein Einschalten einer Lichtquelle, z. B. mehrere LEDs umfassend, im Inneren des Mundhöhlenkörpers, der in einer Mundhöhle einer Person steckt, kann zunächst einmal Licht im Inneren der Mundhöhle einer Person erzeugt werden.

Die Lichtquelle schafft ein gerichtetes Licht, das von dem Mundhöhlenkörper abstrahlt und somit in einer Richtung weg von dem Mundhöhlenkörper leuchtet.

Nachfolgend werden vorteilhafte Ausgestaltungen und Weiterbildungen dargelegt, die für sich gesehen, sowohl einzeln als auch in Kombination, ebenfalls erfinderische Aspekte offenbaren können.

Vorteilhafterweise hat das Mundsteckstück auch eine Energiequelle oder wird von einer ihm zur Verfügung stehenden Energiequelle versorgt. Das Mundsteckstück ist im Betriebszustand mit einer Energiequelle verbunden. Eine Energiequelle kann Teil des Mundsteckstücks sein oder zumindest für das Mundsteckstück zur Verfügung stehen.

Die Lichtquelle ist idealerweise eine Niedervoltlichtquelle, um z. B. das Risiko für einen Nutzer des Mundsteckstücks zu reduzieren. Die Energiequelle ist idealerweise für eine Niedervoltversorgung ausgelegt.

Die Lichtquellen können aus einer oder mehreren LEDs hergestellt sein. Mehrere LEDs können zusammen die Lichtquelle bieten oder bilden.

Als LEDs eignen sich Rot-Grün-Blau-LEDs (RGB-LEDs) bzw. auch dezidierte Blaulicht-LEDs können Teil der Lichtquelle sein oder die Lichtquelle bilden.

Weitere LEDs, die geeignet sind, Teil der Lichtquellen darzustellen, sind UV-Licht-LEDs, z. B. UV-Licht-LEDs eines ganz bestimmten UV-Lichtbandes wie z. B. UV-C. Die Wirkung lässt sich noch steigern, wenn neben dem Licht auch noch eine Abgabe von UV-Strahlung vorgesehen ist, z. B. durch eine Anordnung von LEDs, die Licht abgeben können, und wenigstens einer LED, die eine UV-Strahlung oder einen Laser-Strahl abgeben kann. Das Licht, das aus der Lichtquelle im Mundhöhlenkörper austreten kann, ist vorzugsweise ein gerichtetes Licht, das vorzugsweise in dem posterioren Mundhöhlenbereich eine Beleuchtung sicherstellen kann.

Die Energiequelle kann in einer Ausgestaltung auf einem Spannungsniveau Strom abgeben, der der Versorgungsspannung der Lichtquellen, insbesondere der LEDs, entspricht. Liefert die Energiequelle eine höhere Spannung, so kann mit Hilfe einer elektronischen Schaltung (Tiefsetzsteller, Z-Dioden-Schaltung, Zerhacker mit Transformator oder durch ähnliche Schaltungen) die Spannung auf das Versorgungsspannungsniveau der Lichtquelle reduziert werden.

Zur zeitlichen Steuerung des Betriebs des Mundsteckstücks sollte ein vorteilhaft gestaltetes Mundsteckstück eine Zeitsteuerung umfassen. Durch die Zeitsteuerung kann die Beleuchtungsdauer zeitlich begrenzt werden. Nur während der Beleuchtungsdauer kann die Lichtquelle ein Licht abgeben. Das Mundsteckstück ist zeitlich in seinem Betrieb begrenzt. Ein übermäßiger Lichtkonsum wird so vermieden.

Es ist vorteilhaft, wenn die Dauer der Abgabe von entsprechendem Licht zeitlich beschränkt wird. Eine Zeitsteuerung, durch die die Dauer der Beleuchtung beschränkt wird, trägt zur Schädigungsfreiheit durch das Mundsteckstück bei.

Das Mundsteckstück sollte vorteilhafterweise ein Batteriefach aufweisen. Dieses Batteriefach sollte aber in einem Abschnitt des Mundsteckstücks angeordnet sein, der nicht für die Aufnahme im Mund eines Menschen gedacht ist. Das Batteriefach sollte außerhalb eines menschlichen Körpers verbleiben. Der anzusiedelnde Bereich sollte außerhalb des menschlichen Körpers liegen. In das Batteriefach kann - idealerweise - nach Gusto des Benutzers entweder ein Akkumulator oder eine Batterie eingebaut werden, sofern beide die gleiche Niedervoltversorgungsspannung liefern können.

Die Lichtquelle, die Licht abgeben soll, sollte in einem Wellenlängenbereich zwischen 400 nm und 490 nm liegen. Eine geeignete Wellenlänge ist z. B. bei ungefähr 405 nm. Es hat sich gezeigt, dass eine Wellenlänge um 453 nm positive Effekte hervorrufen kann. Auch eine Wellenlänge im Bereich um 470 nm kann positive Wirkung entfalten.

Ein geeigneter Wellenlängenbereich für UV-Licht ist ein Wellenlängenbereich, der im Bereich um 222 nm liegt. Hierbei sind vorzugsweise solche Wellenlängenbereiche ausgenommen, durch die eine unerwünschte Strahlungsbelastung für körpereigenes Gewebe entsteht, die also als ungünstig angesehen werden, wie eine Wellenlänge von 248 nm. In der medizinischen Fachliteratur sind Meinungen zu finden, dass eine längere Belastung durch Wellen im Wellenlängenbereich um 248 nm zu Krebs bzw. unkontrollierter Zellteilung führen könne.

Wird das Licht aus der Lichtquelle gerichtet durch den Mundhöhlenkörper ausgestrahlt, kann die Wirkung des Mundsteckstücks gesteigert werden, wobei zugleich der Energieverbrauch niedrig gehalten werden kann. Vorzugsweise ist der Spitzenbereich des Mundhöhlenkörpers für eine Abgabe des Lichtes gestaltet.

Ist ein Teil des Mundsteckstücks knopfartig gestaltet, z. B. durch einen knopfartigen Körper, der vorteilhafterweise als Griff fungiert, so kann der knopfartige Körper jenseits der Anlagefläche positioniert sein. Der knopfartige Körper kann flach sein, wodurch die Erstreckung jenseits des Mundes möglichst kurz ist. Die Anlagefläche trennt den Mundhöhlenkörper von dem knopfartigen Körper. Der knopfartige Körper kann als elektrischer Versorgungskörper ausgestaltet sein, z. B. über elektrische Anschlüsse. Als elektrische Anschlüsse können speziell vorgesehene Kabel, aber auch Stecker, an die ein Gegenstecker mit einem entsprechenden Kabel angeschlossen werden kann, vorgesehen sein. Auch kann innerhalb des knopfartigen Körpers ein Batteriefach existieren, in das eine Batterie als Energiequelle für den Betrieb der Lichtquelle eingebaut werden kann.

Vorteilhaft ist es, wenn das Mundsteckstück bzw. Teile desselben, z. B. der Mundhöhlenkörper, nachgiebig ausgestaltet ist. Auch ist es vorteilhaft, wenn das Mundsteckstück flüssigkeitsdicht ausgestaltet ist. Das Mundsteckstück kann, insbesondere aufgrund geeigneter Materialwahlen, sowohl nachgiebig als auch flüssigkeitsdicht ausgestaltet sein. In einigen Gestaltungen mag es ausreichen, wenn das Mundsteckstück nur nachgiebig ist. In anderen Ausgestaltungen mag es ausreichen, wenn das Mundsteckstück flüssigkeitsdicht ausgestaltet ist. Als Materialien für die Schaffung des Mundsteckstücks bieten sich Silikone oder Latex an. Das Mundsteckstück, zumindest aber sein Mundhöhlenkörper kann entweder aus einer Kunststoffhülle hergestellt sein oder aus einer Latex-Hülle.

Das Mundsteckstück kann von außen betrachtet wie ein zwei Halbschalen umfassender größerer Körper mit einem dahinter angeordneten, kleineren Körper ausgestaltet sein. Der oval gestaltete Querkörper, der Mundhöhlenkörper, ist idealerweise symmetrisch gestaltet. Vorzugsweise ist eine Langachse des Ovals entlang einer Bissebene ausgerichtet. Es ist möglich, solche Raumgestaltungen zu wählen, dass sowohl eine dentogingivale als auch eine linguale Anlagerung des Mundhöhlenkörpers im Mund des das Mundsteckstück nutzenden Menschen gegeben ist. Ein Körper, der in Bezug auf seine Ober- und Unterschale symmetrisch gestaltet ist, erleichtert die geeignete Platzierung im Mund. Auch lässt sich so ein Gegenstand leichter herstellen.

Ist das Mundsteckstück nachgiebig gestaltet, so kann es so nachgiebig gestaltet sein, dass es formadaptiv im Mund anliegen kann. Eine formadaptive Oberfläche folgt den Druckverhältnissen im Mund, z. B. einem Druck durch eine Zunge oder einem Unterdruck durch Atemvorgänge. Die formadaptive Oberfläche ist zur Nachbildung der Mundhöhle eines Nutzers sinnvoll.

Die Wirkung, dass durch das Mundsteckstück Keime reduziert werden, kann dadurch gesteigert werden, dass der Mundhöhlenkörper verschiebbar ausgestaltet ist. Der Mundhöhlenkörper kann in einer vorteilhaften Ausgestaltung gegenüber der Anlagefläche bzw. der Auflage räumlich bewegt werden. Der Mundhöhlenkörper kann z. B. ausziehbar gestaltet sein. Ist der Mundhöhlenkörper verlängerbar, so kann das Licht aus dem Mundhöhlenkörper möglichst gut an die zu bestrahlende Stelle, wie z. B. den Rachenraum eines Menschen, gelangen. Die Mundhöhle verschiedener Menschen ist unterschiedlich groß - Kinder haben z. B. eine sehr viel kleinere Mundhöhle als Erwachsene. Hierfür können Mundsteckstücke unterschiedlicher Größe bereitgestellt werden oder es wird ein Universal-Mundsteckstück bereitgestellt, bei dem der Mundhöhlenkörper in seiner Größe, insbesondere Längserstreckung, veränderbar ist. Es ist also die Länge des Mundsteckstücks durch eine verschiebliche Anordnung des Mundhöhlenkörpers veränderbar.

Sollte die Länge des Mundsteckstücks nicht passen, z. B. weil ein Mund eines Nutzers des Mundsteckstücks kürzer ist als der Mundhöhlenkörper lang ist, so kann durch wenige Handgriffe aus einem ersten Mundsteckstück ein zweites, passenderes Mundsteckstück werden. Hierzu kann der Mundhöhlenkörper von dem Mundsteckstück getrennt werden. Eine besonders einfache Methode des Trennens ist dadurch geschaffen, dass durch ein Herausbiegen des Mundhöhlenkörpers bzw. seines Endes aus Befestigungsnasen die beiden markantesten bzw. größten Teile des Mundsteckstücks zu trennen sind (sogenannte Klippnasen). Die Befestigungsnasen können im Randbereich eines blockartigen Anschlusskörpers des Mundsteckstücks vorhanden sein. Ein Ende des Mundhöhlenkörpers kann als flache Platte ausgestaltet sein. Die flache Platte bildet eine Seite des hinteren Körpers des Mundsteckstücks. Die Platte wird durch die Befestigungsnasen gehalten.

Ist der erste Mundhöhlenkörper so lang, dass er einen Würgereiz bei einem Menschen hervorrufen kann, so kann durch Austausch des Mundhöhlenkörpers mit einem kurzen stabartigen Licht- bzw. Strahlenspender ein Mundsteckstück geschaffen werden, das besser auf die Anatomie des Mundraums des Nutzers angepasst ist.

Sind die Klippnasen genau entgegengesetzt zueinander orientiert, so kann durch zwei Bewegungen, idealerweise zeitgleich auszuführende Bewegungen, ein Block an dem Mundhöhlenkörper so in seiner Breite oder Höhe verändert werden, dass er sich von dem Körper, der außerhalb des Mundes verbleiben soll, trennt. Solche Bewegungen können als verengende bzw. zusammendrückende Bewegungen oder als aufspreizende Bewegungen vorgesehen sein.

Die vorteilhaften Weiterbildungen können auch wie folgt vorgestellt werden.

Werden LED-Stripes als Lichtquellen verwendet, so kann der Mundhöhlenkörper sehr schmal, z. B. weniger als 5 cm in seiner Breite, gestaltet sein. Werden Stripes verwendet, die aus LEDs mit einer Breite von 3 mm aufgebaut sind, so kann der stab- oder zapfenähnliche Mundhöhlenkörper so dünn sein, dass er gerade einmal 5 mm beträgt. Je schmaler der Mundhöhlenkörper ausgeführt ist, desto geringer fällt seine hindernde oder beeinflussende Eigenschaft ins Gewicht.

Idealerweise hat das Mundsteckstück eine eigene Energiequelle, z. B. in sich integriert bzw. eingebaut. Die Energiequelle ist, wenn es sich um einen Akkumulator handelt, nach einigen Betriebsstunden wieder aufzuladen. Hierzu bietet sich ein integrierter, insbesondere standardisierter Anschluss. Z. B. kann ein USB-Anschluss wie ein USB-C-Anschluss oder ein USB 3.0-Anschluss die Buchse für den Anschluss eines Kabels zur Stromversorgung und zum Aufladen des integrierten Akkumulators in den hinteren Teil des Mundsteckstücks eingearbeitet sein. In dem Bereich, der nicht für die Aufnahme im Mund bestimmt ist, kann eine Buchse für den Anschluss eines Kabels vorgesehen sein.

Eine Lichtquelle, die sich im Inneren des Mundhöhlenkörpers befindet, kann sich aus einem Satz verschiedener LED-Typen zusammensetzen. Mehrere LEDs, insbesondere LEDs des Typs Stripe-LED, können eine Beleuchtungsgruppe bilden, die benachbart zu anderen LEDs angeordnet mit den weiteren LEDs eine Beleuchtungsgruppe bilden, z. B. mehrere Blaulicht-LEDs mit einer LASER-LED und mit einer UV-LED. So können Sätze von LEDs zusammengestellt werden, die sich aus LEDs wenigstens zwei verschiedener Typen zusammensetzen.

Mehrere, insbesondere typgleiche LEDs, z. B. die Blaulicht-LEDs, können auf einem LED-Träger angeordnet sein. Werden die LEDs entlang des Mundhöhlenkörpers für unterschiedliche Lichtausstrahlungen platziert, z. B. durch einen Betrieb mit verschiedenen Leistungen oder z. B. durch dichtere oder weiter entferntere Platzierungen der LEDs, können Bereiche mit helleren und Bereiche mit weniger hellen Ausleuchtungen erzeugt werden. Durch vorzugsweise unterschiedliche Lichtdichten in verschiedenen Abschnitten des Mundhöhlenkörpers sind hellere und dunklere Bereiche herstellbar. So ist es möglich, von einer LED zu einer anderen LED mit untereinander abweichenden Versorgungsströmen zu operieren. Auch ist es möglich, in einem ersten Bereich LEDs dichter anzuordnen als in einem zweiten Bereich. In dem zweiten Bereich können die LEDs weiter beabstandet sein. In einem ersten Bereich sind die LEDs dichter platziert als in einem zweiten Bereich. Der zweite Bereich hat LEDs, die weiter beabstandet platziert sind als in dem ersten Bereich. Entlang des Mundhöhlenkörpers können LEDs unterschiedlich dicht platziert sein. Wird in einem Rachenbereich oder in einem posterioren Mundhöhlenbereich eine höhere Helligkeit benötigt, können die LEDs, die das Licht für jenen Bereich ausstrahlen sollen, mit einem stärkeren Versorgungsstrom versorgt werden.

Ist das Mundsteckstück mit einer Zeitsteuerung ausgestattet, so können die Betriebsphasen der Lichtquelle gesteuert werden. Ist ein Temperatursensor in dem Mundsteckstück, insbesondere im Mundhöhlenkörper integriert, so ist eine zusätzliche Sicherheitseinrichtung vorhanden. LEDs, die Licht oder eine elektromagnetische Welle abgeben sollen, können nur dann ausstrahlen, wenn sowohl die Zeitsteuerung es zulässt als auch der Temperatursensor keine erhöhte oder unzulässig hohe Temperatur registriert. Mit einer Zeitsteuerung für unterschiedliche Lichtquellen oder unterschiedliche Beleuchtungskörper, z. B. für einen LED-Stripe mit Blaulicht-LEDs und für eine LASER-LED, kann der Stripe über eine andere Dauer betrieben werden als die LASER-LED. Ähnliche Abweichungen können zwischen Blaulicht-LEDs und UV-(Licht-)LEDs eingestellt werden, z. B. kann die eine UV-LED oder es können die mehreren UV-LEDs nur kürzer betrieben werden als die Blaulicht-LEDs.

Eine LED, die eine Wellenfront oder eine Strahlung jenseits des sichtbaren Wellenspektrums aussendet, ist z. B. eine UV-Licht-LED. Eine Blaulicht-LED kann länger betrieben werden als eine UV-Licht-LED oder eine sonstige LED, die eine Wellenfront jenseits des sichtbaren Wellenspektrums aussendet, wenn Zellschäden möglichst unterbunden bleiben sollen.

Durch eine Temperaturüberwachung, z. B. mittels des oben beschriebenen Temperatursensors, kann sichergestellt werden, dass nur Licht bzw. eine elektromagnetische Welle von dem Mundsteckstück abgegeben wird, wenn das Mundsteckstück, insbesondere sein Mundhöhlenkörper, unterhalb einer Temperaturschwelle, wie z. B. 38 °C, liegt.

Ist in dem Mundsteckstück ein Akkumulator oder eine Batterie eingebaut, insbesondere auf der Steuerungsplatine eingelötet, so kann das Mundsteckstück auch ohne Anschluss an eine Netzversorgung betrieben werden. Ein mechanisch fest verbundener Energiespeicher wie ein Akkumulator, z. B. durch eingelötete Lötfahnen des Akkumulators, kann zur besseren Gleichgewichtslage im Mund des Nutzers beitragen.

Der Teil des Mundsteckstücks, der außerhalb des Mundes verbleiben soll, kann als Halteknopf, kastenförmiger Griff oder blockartiger Körper ausgestaltet sein, um ein Gegengewicht zum Mundhöhlenkörper zu bilden und darüber hinaus eine Angriffsstelle zu bieten, durch die der Mundhöhlenkörper beliebig im Mund bewegt werden kann.

Vorteilhafterweise hat das Mundsteckstück an seinem seitlichen Bereich einen Einschaltknopf. Der Einschaltknopf kann mit einem Einschalttaster kooperieren. Der Einschalttaster kann z. B. im Inneren des Körpers platziert sein. Die Steuerung des Mundsteckstücks kann so gestaltet sein, dass der Einschalttaster nur zu ganz bestimmten Zeitpunkten oder nach ganz bestimmten Zeitdauern wieder betätigbar ist bzw. erneut Signale zur Verfügung stellen kann. Der Einschalttaster kann ein zeitlich verriegelnder Taster sein. Nur nach bestimmten Zeiten steht er wieder zu Verfügung. Nur wenn eine gewisse Zeit, wie z. B. 30 Minuten, abgelaufen bzw. vorbei sind, kann ein neuer Schaltimpuls ausgelöst werden.

Die zuvor beschriebenen Ausgestaltungen des Mundsteckstücks können auch wie folgt zusammengefasst werden.

Ein Mundsteckstück kann derart gestaltet sein, dass es wie ein Schnuller im vorderen Mundraum, z. B. an Zunge und Gaumen, anliegen kann. Der nicht für die Anlage vorgesehene Bereich des Mundsteckstücks, genauer des transluzenten Mundhöhlenkörpers, ist für die Abgabe von Licht wenigstens in einen posterioren Mundhöhlenbereich aus wenigstens einer Lichtquelle, die im Inneren des Mundhöhlenkörper angeordnet ist, vorgesehen.

Gem. einem vorteilhaften Verfahren lässt sich das Mundsteckstück mit einem solchen Mundhöhlenkörper herstellen, der eine abgestimmte Länge für eine ganz bestimmte Lage oder Position in einer Mundhöhle eines Nutzers hat. Der ausgewählte Mundhöhlenkörper ist aus einem Satz von Mundhöhlenkörpern auswählbar. Der Mundhöhlenkörper ist über einen Steckverbinder an eine Steuerplatine in einem blockartigen Körper des Mundsteckstücks anzuschließen. Durch Verwendung eines solchen Mundsteckstücks ist es möglich, die Keimzahl, insbesondere im Rachenbereich eines Menschen, durch Lichtbestrahlung mit Licht keimtötend wirkender Wellenlängen zu reduzieren. So ist es möglich, ein tragbares Desinfektionsgerät an der Hand zu haben.

Weitere vorteilhafte Ausgestaltungen sollen nachfolgend vorgestellt werden.

Die beabsichtigte Keimzahlreduktion kann noch weiter verbessert werden, indem ein lichtaktivierbarer Inhibitor, Wirkstoffverstärker, Wirkverstärker oder Energieumsetzer in den Mund eingebracht wird, z. B. über das Mundsteckstück oder über eine Mundspülung. Werden Zusatzstoffe, wie z. B. ein Reaktant, zusätzlich in den Mundraum gegeben, so kann die Wirkung des Mundsteckstücks noch verbessert werden. Der Reaktant, der Inhibitor, der Wirkstoffverstärker bzw. der Wirkverstärker können auf die Wellenlänge des von dem Mundsteckstück ausgestrahlten Lichts bzw. der von dem Mundsteckstück abgestrahlten elektromagnetischen Welle abgestimmt sein. Der lichtaktivierbare Inhibitor kann auch kurz als Lichtinhibitor bezeichnet werden. Der Lichtinhibitor reagiert idealerweise auf genau die Wellenlänge, die von dem Mundsteckstück ausgesendet wird. Beispielsweise kann der Inhibitor ein Hemmstoff sein, der Stoffwechselaktivitäten verlangsamt. Der Inhibitor kann auch ein Hemmstoff sein, der die Vermehrung von Bakterien oder Viren verlangsamt oder unterdrückt. Auch kann der Inhibitor so ausgewählt sein, dass er Moleküle oder eine Molekülmischung umfasst, die das Eindringen von Viren in Zellen unterdrückt. Ein Beispiel für einen Inhibitor ist ein Enzym. Ein Enzym, das durch blaues Licht aktivierbar ist, ist z. B. ein speziell gestaltetes Enzym des Typs Adenylatzyklase (Adenylylcyclasen). Ein anderes Beispiel für einen Inhibitor ist ein Ion, wie ein Silberion. Durch eine geeignete Lichtwellenlänge kann ein Inhibitor durch eine Freisetzung im Mundraum, z. B. im Speichel, aktivierbar sein, indem z. B. eine chemische Bindung an einen Inhibitorträger aufgebrochen wird, insbesondere photochemisch gebrochen wird. Ein weiteres Beispiel ist Silbernitrat, mit dem Wucherungen und Geschwüre im Rachenbereich und im hinteren Mundbereich behandelt werden können. Eine andere Möglichkeit der Aktivierung eines Inhibitors besteht in der photochemischen Abspaltung einer molekularen Schutzeinheit, z. B. eines Moleküls. Außerdem kann ein Inhibitor durch Aufnahme elektromagnetischer Energie in einen elektronisch angeregten Zustand verbracht werden, der ein Aktivierungszustand ist. Durch die Vorgabe einer Lichtintensität und einer Bestrahlungsdauer ist eine bereitgestellte Menge des reaktiv oder katalytisch wirkenden Inhibitors genau dosierbar.

Das Mundstück weist vorzugsweise eine Steuereinheit auf, durch die eine Abgabe einer Lichtdosis, insbesondere einer auswählbaren Lichtwellenlänge, z. B. sensorisch, kontrollierbar ist.

Besonders vorteilhaft ist ein Mundsteckstück, das den Inhibitor, Wirkstoffverstärker, Wirkverstärker oder Energieumsetzer dosiert im Mundraum abgeben kann, z. B. über einen Docht oder über eine Dosierkanüle.

Wie gesagt, bieten sich zur Erzeugung des Lichts unterschiedliche Wellenlängenbereiche an. Ein besonders bevorzugter Wellenlängenbereich liegt in einem Bereich zwischen 400 nm und 480 nm, wobei sich z. B. besonders ausgewählte Wellenlängen wie 405 nm, 453 nm oder 470 nm als vorteilhaft herausgestellt haben. Daneben ist es vorteilhaft, wenn ein UV-Licht-Wellenlängenbereich ebenfalls von dem Mundsteckstück abgedeckt werden kann. Ein solcher Bereich kann z. B. ein UV-C-Wellenlängenbereich sein, wie z. B. zwischen 100 nm und 280 nm. Hierbei versteht ein Fachmann, dass von den breit angegebenen Wellenlängenbereichen wiederum einzelne, engere Wellenlängenbereiche ausgeschlossen sein können, um insbesondere schädigende Wirkungen für menschliche Organismen auszuschließen.

Der Weg des Lichts kann zur Erzeugung eines besonderen visuellen Effekts genutzt werden, der z. B. zu besonderen Anlässen wie Karneval oder einer Feier eine positive, zumindest aber markante Erscheinung hervorrufen kann.

Durch eine anschließende Nutzung der Reflexion bzw. der Streuung zumindest eines Teils des Lichts in Richtung auf den Mundbereich, insbesondere an einer Rachenwand im Mundraum der Person, kann das Licht auf die Lippen oder die Mundpartie der Person scheinen und hierdurch eine Verfärbung hervorrufen. Eine Reflektion von Licht kann z. B. an Zähnen oder Zahnkronen erfolgen. Das Licht, das vorzugsweise reflektiertes Licht ist, tritt aus der Mundhöhle über die Lippen aus. Mithilfe von aufgetragenen fluoreszierenden Hautcremes lassen sich in Verbindung mit blauem Licht oder UV-Licht z. B. Lichteffekte auf der Haut generieren.

Das Mundsteckstück ist als mobiles Desinfektionsgerät gestaltet, bei dem mittels Licht Keime im Mund, insbesondere im Rachen, eines Lebewesens, wie im Rachen eines Menschen, abgetötet werden können. Wird mit einer Wellenlänge gearbeitet, die an Körperteilen wie Zähnen reflektiert werden kann, so kann die Wirkung des ausgestrahlten Lichts gesteigert werden.

Mit dem Verfahren kann eine Verfärbung durchgeführt werden. Diese Verfärbung kann bedarfsabhängig, insbesondere nur in besonderen temporären Phasen, gewählt werden, z. B. durch ein kurz aufeinander folgendes Ein- und Ausschalten.

Besonders markant kann es in bestimmten Umständen sein, wenn eine Blaulichtverfärbung eines Mundbereichs einer Person hervorgerufen wird. So können z. B. die Lippen einer Person durch das Mundsteckstück nur vorrübergehend visuell wahrnehmbar umgefärbt werden (von kurzer zeitlicher Dauer).

Die Hülle des Mundhöhlenkörpers kann mit Geschmacks- und/oder Aromastoffen, z. B. einem Erdbeergeschmack, veredelt sein. Dadurch lässt sich die Akzeptanz steigern. Wenn der Mundhöhlenkörper nicht mehr nur nach Silikon schmeckt, sondern nach einer angenehmen Geschmacksnote, wie Whiskey oder Trüffel, steigert es die Bereitschaft, das Mundsteckstück in den Mund einzuführen.

Der Mundhöhlenkörper kann mit einer zweigeteilten Spitze ausgestattet sein, sodass ein Ende des Mundhöhlenkörpers das Gaumenzäpfchen im Mund, die Uvula, seitlich passieren kann.

Die zuvor dargestellten Kombinationen und Ausführungsbeispiele lassen sich auch in zahlreichen weiteren Verbindungen und Kombinationen betrachten.

Neben den zuvor dargestellten Varianten und Ausführungsformen ist es auch vorstellbar, dass das Mundsteckstück eine Kombination unterschiedlicher Lichtquellen beinhaltet, z. B. eine UV-Lichtquelle neben einer Blaulicht-Lichtquelle. Ist die Steuerung des Mundsteckstücks etwas raffinierter gestaltet, so kann zwischen dem Betrieb der UV-Lichtquelle und der Blaulichtquelle mit einem zeitlichen Muster hin- und hergeschaltet werden. Hierbei ist es besonders vorteilhaft, wenn die Phasen der einen Lichtbestrahlung und die Phasen der anderen Lichtbestrahlung nicht gleich lang, sondern in Abhängigkeit des zu erreichenden Ziels in unterschiedlichen prozentualen Anteilen betrieben werden.

Überraschenderweise hat sich gezeigt, dass mit dem Corona-Virus infizierte Menschen, die Halsschmerzen und andere corona-typische Krankheitssymptome aufweisen, durch wenige Behandlungen bzw. Eigenbehandlungen mit einem zuvor beschriebenen Mundsteckstück geheilt werden können. So hat sich in einem Fall gezeigt, dass eine Person, die Krankheitssymptome zeigte und einen positiven Corona-Test vorweisen musste, nach nur einer einstelligen Anzahl Behandlungen, z. B. nach fünf Behandlungen, geheilt war. Ein zweiter Corona-Test nach nur fünf Tagen fiel negativ aus. Die Person hatte nach dem ersten positiven Corona-Test und bis zu dem zweiten Corona-Test einmal täglich während der Dauer von fünf Tagen ein Mundsteckstück in ihren Mund eingeführt und für ca. 15 Minuten das Mundsteckstück betrieben bzw. blau leuchten lassen.

Ein autonom versorgtes, d. h. mit einem Speicher für elektrische Energie ausgestattetes Mundsteckstück kann auch als mobiles Gerät von Personen in öffentlichen Gebäuden und Verkehrsmitteln mitgeführt werden. Sollte eine Person feststellen, dass sie sich einer erhöhten Gefährdungslage aussetzen musste, z. B. aufgrund von unterschrittenen Mindestabständen oder z. B. aufgrund von unerwünschten Ereignissen (wie Anhusten, wie Anspucken, wie Anhauchen durch einen Dritten), kann sie unverzüglich Gegenmaßnahmen ergreifen, indem sie sich das Mundsteckstück in den Mund steckt und für eine gewisse Dauer, z. B. für 10 Minuten oder 15 Minuten einschaltet, um insbesondere eingetretene oder eintretende Keime durch Licht abzutöten.

### Figurenkurzbeschreibung

Die vorliegende Erfindung kann noch besser verstanden werden, wenn Bezug auf die beiliegenden Figuren genommen wird, die beispielhaft besonders vorteilhafte Ausgestaltungsmöglichkeiten darlegen, ohne die vorliegende Erfindung auf diese einzuschränken. Mit anderen Worten,
- Figur 1: zeigt ein Referenzbeispiel eines Mundsteckstücks,
- Figur 2: zeigt in einer perspektivischen Ansicht eine erste Ausführungsform eines erfindungsgemäßen Mundsteckstücks,
- Figur 3: zeigt eine erste Seitenansicht der Ausführungsform nach Figur 2,
- Figur 4: zeigt eine zweite Seitenansicht der Ausführungsform nach Figur 2,
- Figur 5: zeigt eine Ansicht mit Blickrichtung auf einen Kastenkörper gem. der Ausführungsform nach Figur 2,
- Figur 6: zeigt eine Explosionsdarstellung einer zweiten Ausführungsform eines erfindungsgemäßen Mundsteckstücks,
- Figur 7: zeigt in perspektivischer Darstellung eine Variante eines LED-Trägers ohne Mundschutz mit weiteren Bauteilen eines Mundsteckstücks gem. der Ausführungsform nach Figur 6,
- Figur 8: zeigt das Innere des Gehäuses mit dem Batteriefach gem. der Ausführungsform nach Figur 6,
- Figur 9: zeigt in einer Schnittansicht einen Schnitt durch das Mundsteckstück gem. der Ausführungsform nach Figur 6,
- Figur 10: zeigt, wie eine dritte Ausführungsform eines erfindungsgemäßen Mundsteckstücks in einen Mundraum einer Person eingesetzt werden kann,
- Figur 11: zeigt eine vierte Ausführungsform eines erfindungsgemäßen Mundsteckstücks,
- Figur 12: zeigt eine fünfte Ausführungsform eines erfindungsgemäßen Mundsteckstücks,
- Figur 13: zeigt eine sechste Ausführungsform eines erfindungsgemäßen Mundsteckstücks,
- Figur 14: zeigt einen LED-Streifen auf einer Platine, die mit dem LED-Träger nach Figur 13 zu einem Mundsteckstück zusammenfügbar ist,
- Figur 15: zeigt eine achte siebte Ausführungsform eines erfindungsgemäßen Mundsteckstücks und
- Figur 16: zeigt eine achte Ausführungsform eines erfindungsgemäßen Mundsteckstücks.

### Figurenbeschreibung

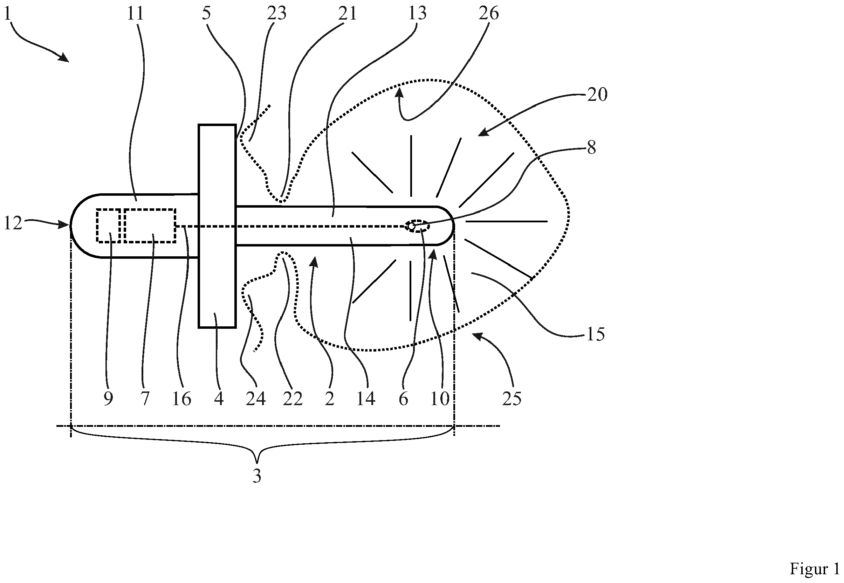

In Figur 1 ist ein Referenzbeispiel eines Mundsteckstücks 1 grob schematisch dargestellt. Es besteht aus einem knopfartigen Körper 11, der als Griff dient, einer Anlagefläche 4, die auch als Schild bezeichnet werden kann, und einem Mundhöhlenkörper 2.

Das Mundsteckstück 1 kann derart gestaltet sein, dass es wie ein Schnuller im vorderen Mundraum, z. B. an Zunge 25 und Gaumen 26, anliegen kann. Der nicht für die Anlage vorgesehene Bereich des Mundsteckstücks 1, genauer des Mundhöhlenkörpers 2, ist für die Abgabe von Licht 15 aus einer Lichtquelle 6, die im Mundhöhlenkörper 2 angeordnet ist, vorgesehen. Mit einem solchen Mundsteckstück 1 ist es möglich, die Keimzahl, insbesondere im Rachenbereich eines Menschen, durch Lichtbestrahlung zu reduzieren.

In Versuchen zeigte sich, dass nach einer Bestrahlungsdauer von 15 Minuten die Keimzahl reduziert war.

Im knopfartigen Körper 11 sind in der dargestellten Ausführung eine Energiequelle 7, z. B. eine Batterie oder ein Akkumulator, und eine Zeitsteuerung 9 untergebracht. Es ist auch ein elektrischer Anschluss 12 vorgesehen, um z. B. einen eingesetzten Akkumulator zu laden, ohne dass er entnommen werden muss.

Im Mundhöhlenkörper 2 ist als Lichtquelle 6 eine LED 8 vorgesehen. Es können auch mehrere LEDs vorhanden sein, oder auch andere Lampen. Die Lichtquelle 6 und die Energiequelle 7 sind über Kabel 16 miteinander verbunden.

Im vorderen Bereich, d. h. im Bereich der Spitze des Mundhöhlenkörpers 2, ist ein Lichtabgabebereich 10 vorgesehen. Dieser Bereich des Mundhöhlenkörpers 2 ist transluzent, sodass das von der Lichtquelle 6 abgestrahlte Licht 15 austreten kann. Ist nur der vordere Bereich transluzent, so hat dies den Vorteil, dass gezielt der dem Rachen zugewandte Bereich der Mundhöhle bestrahlt werden kann, wohingegen der Bereich der Mundhöhle 20, der hinter den Zähnen liegt, unbestrahlt bleibt. Der Mundhöhlenkörper 2 liegt im in den Mund eingesetzten Zustand auf der Zunge 25 auf und an dem Gaumen 26 an und bestrahlt den Rachenraum (nicht dargestellt).

Der Mundhöhlenkörper 2 ist in der dargestellten Ausführungsform stabförmig und im Querschnitt rotationssymmetrisch. Er besteht aus zwei Halbschalen, der ersten Halbschale 13 und der zweiten Halbschale 14. Der Mundhöhlenkörper 2 kann jedoch auch asymmetrisch gestaltet sein, wie es beispielsweise von handelsüblichen Babyschnullern bekannt ist. In allen Fällen ist zumindest der vordere Bereich, d. h. der Bereich, der von der Auflage 5 des Mundsteckstücks 1 am weitesten entfernt ist, transluzent.

Aus dem Referenzbeispiel in Figur 1 ergibt sich, wie das Mundsteckstück 1 im Mund eingesetzt ist. Mit Hilfe des knopfartigen Körpers 11 wird der Mundhöhlenkörper 2 in den Mund eingeführt. Die Anlagefläche 4, das Schild, dient dabei als Anschlag. Die Anlagefläche 4 erstreckt sich ungefähr rechtwinklig zur Längserstreckung 3 des Mundsteckstücks 1. Entsprechend liegt die Auflage 5 im eingesetzten Zustand an den Lippen 23, 24 an. Der Mundhöhlenkörper 2 ist zwischen den Zähnen 21, 22 hindurchgeführt und ragt in die Mundhöhle 20 hinein.

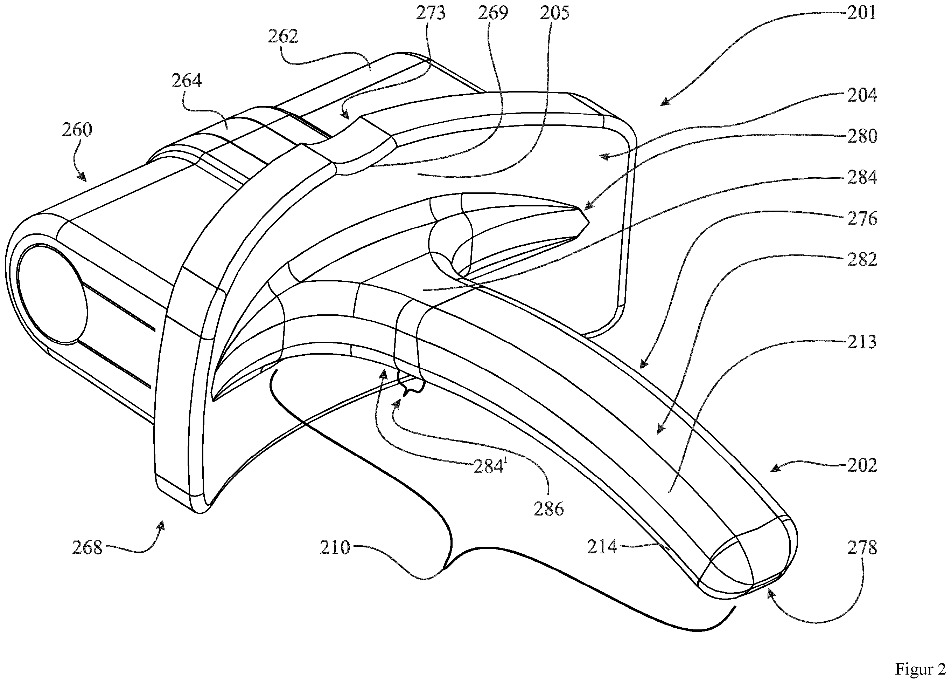

Die Figuren Figur 2 bis Figur 5 zeigen verschiedene Ansichten eines ersten Ausführungsbeispiels eines Mundsteckstücks 201.

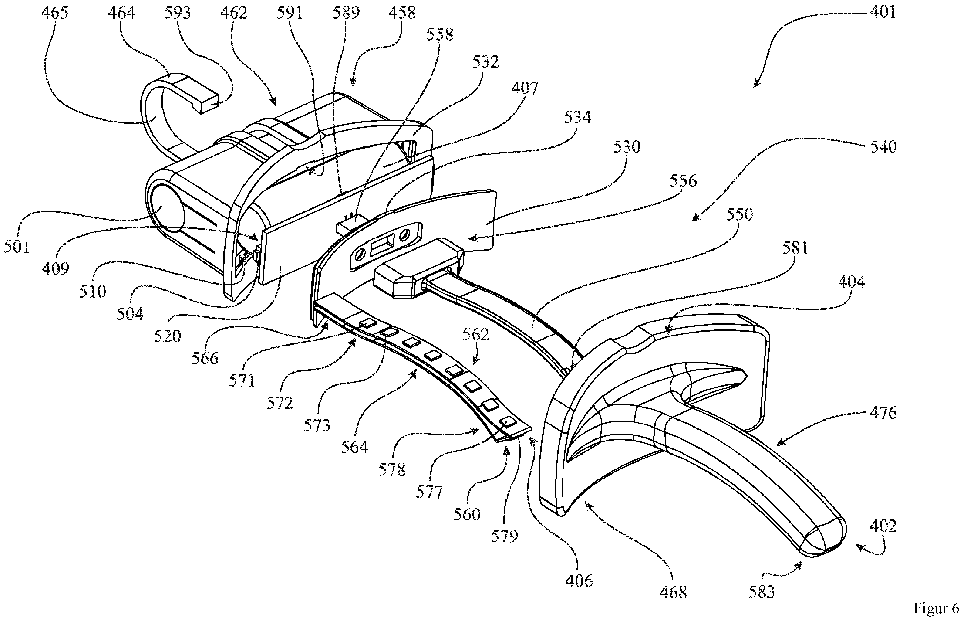

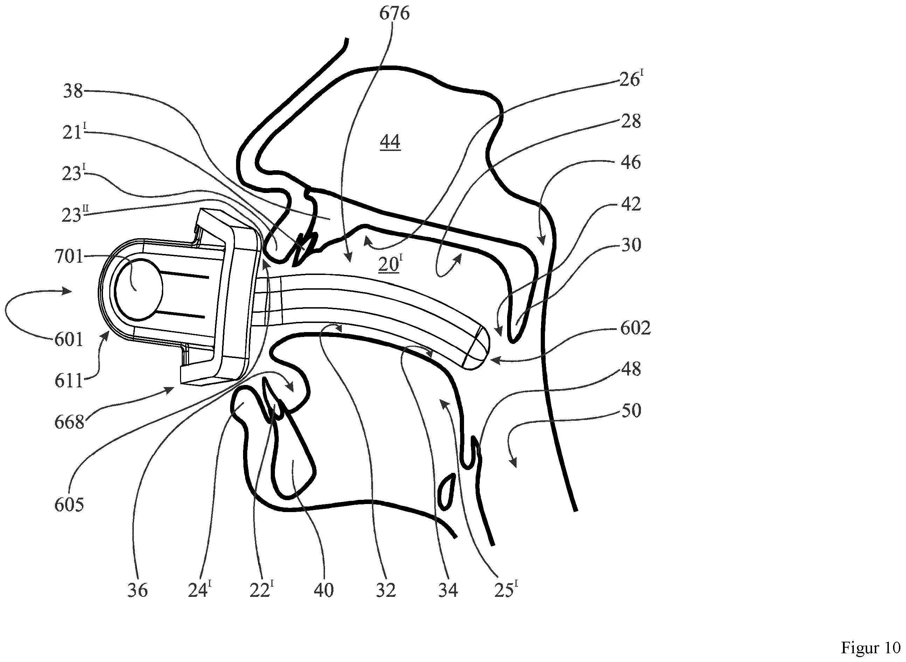

Das in Figur 2 gezeigte Mundsteckstück 201 ist aus einem Mundhöhlenkörper 202 und einem kastenartigen Körper 260 zusammengesetzt. Zwischen dem Mundhöhlenkörper 202 und dem kastenartigen Körper 260 befindet sich ein Mundschutz 268, der eine zylindermantelartig gewölbte Anlagefläche 204 hat. Die Anlagefläche 204 ist konkav dem Mundhöhlenkörper 202 zugewandt. Die Anlagefläche 204 wirkt abdeckend und wölbt sich quer zum Mundhöhlenkörper 202. Die Anlagefläche 204 bildet eine Auflage 205 für einen Gesichtsbereich eines Anwenders des Mundsteckstücks 201. Ein Lippenbezugspunkt 269 dient als Bezugspunkt für eine Eindringtiefe des Mundhöhlenkörpers 202 in eine Mundhöhle (für Details zur Eindringtiefe vgl. die Mundhöhle 20 in Figur 1 bzw. die Mundhöhle 20^{I} in Figur 10). Der Lippenbezugspunkt 269 und eine Dehnungsmulde 273 fallen örtlich zusammen. Durch die Dehnungsmulde 273 wird eine Abnahme bzw. ein Abziehen des Mundschutzes 268 von dem kastenartigen Körper 260 erleichtert. Der Mundschutz 268 ist fest mit dem Mundhöhlenkörper 202 verbunden. Der kastenartige Körper 260 hat eine Fläche, die als Griff 264 genutzt werden kann und die aus dem Gehäuse 262 hervorsteht. Vorteilhafterweise ist der Griff 264 durchsichtig und lässt Licht als eine Art und Weise der Betriebsanzeige austreten, sodass auch außerhalb des Mundes zu erkennen ist (z. B. durch einen Blick des Nutzers des Mundsteckstücks 201 nach unten), ob das Mundsteckstück 201 einwandfrei arbeitet. Das Gehäuse 262 dient u. a. zur Aufnahme eines Akkumulators und einer Steuerplatine in seinem Inneren (vgl. hierzu auch die Ausführungsvariante nach Figur 6). Das Gehäuse 262 ist so groß gestaltet, dass es als Griff 264 dienen kann und zugleich genug Platz für die elektrischen und elektronischen Bauteile zur Ansteuerung des transluzenten Körpers 276 bietet. Der Mundhöhlenkörper 202 ist durch eine erste Halbschale 213 und durch eine zweite Halbschale 214 - von außen betrachtet - abgeschlossen, die gemeinsam eine Kunststoffhülle 282 des Mundhöhlenkörpers 202 darstellen. Die erste bzw. obere Halbschale 213 ist fest mit der zweiten bzw. unteren Halbschale 214 verbunden. Der Mundhöhlenkörper 202 erstreckt sich in Gestalt eines bogenförmigen Stiels 278. Der bogenförmige Stiel 278 ist als transluzenter Körper 276 realisiert, der einen Lichtabgabebereich 210 hat. Der Lichtabgabebereich 210 erstreckt sich - in der Ausführungsvariante nach Figur 2 - über die gesamte Länge des Mundhöhlenkörpers 202. Der Lichtabgabebereich 210 weist einen Verlängerungsbereich 286 auf, der zur Verfügung steht, um in dem Verlängerungsbereich 286 den bogenförmigen Stiel 278 über eine vorgegebene Strecke auseinanderziehen zu können (im Sinne eines verlängerbaren Stiels 278). Der Lichtabgabebereich 210 ist somit in seiner Länge anpassbar. Die beiden Halbschalen 213, 214 sind eine Fortsetzung der Zahnstützflächen 284, 284^{I}, die auch als Schneidezahnbissflächen bezeichnet werden können. An die Zahnstützflächen 284, 284^{I} schließt sich in eine Richtung auf die Anlagefläche 204 hin ein Lippenanschlusskörper 280 an. Der Lippenanschlusskörper 280 besitzt ein Mundöffnungsprofil. Der Lippenanschlusskörper 280 erleichtert eine Abdichtung einer Mundöffnung unmittelbar an der Anlagefläche 204, um z. B. Speichel zurückzuhalten. Der Lippenanschlusskörper 280 erhebt sich von der Anlagefläche 204 und ist fest mit der Anlagefläche 204 und mit der oberen Zahnstützfläche 284 und der unteren Zahnstützfläche 284^{I} verbunden.

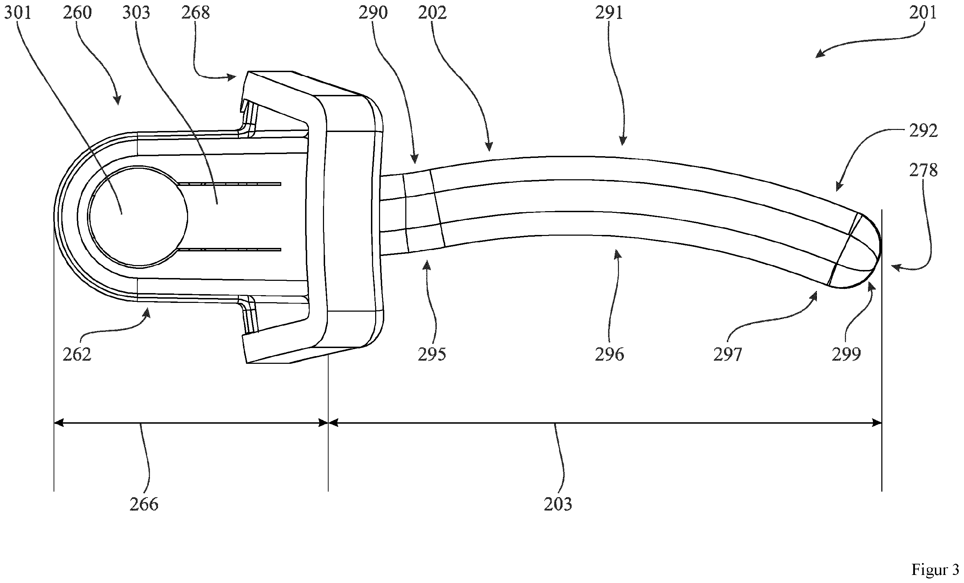

Figur 3 stellt das in Figur 2 gezeigte Mundsteckstück 201 in einer ersten Seitenansicht dar. Der bogenförmige Stiel 278 weist eine Längserstreckung 203 auf. Zur Anpassung an anatomische Gegebenheiten (siehe die anatomischen Gegebenheiten, die in Figur 10 dargestellt sind) wird der bogenförmige Stiel 278 durch mehrere bogenförmige Verläufe gebildet, die voneinander abweichende Krümmungsradien haben. Der Stiel 278 setzt sich aus einer größeren Anzahl ineinander übergehender Bögen bzw. Krümmungen zusammen. Ein erster Stielbogen 290 ist ein gaumenseitiger Stielansatzbogen. Der erste Stielbogen 290 dient der Aufnahme von oberen Schneidezähnen eines das Mundsteckstück 201 umschließenden Mundes (nicht dargestellt). Der erste Stielbogen 290 geht über in einen zweiten Stielbogen 291, der als gaumenseitiger Stielmittenbogen ausgebildet ist. Der zweite Stielbogen 291 folgt dem harten Gaumen. Der zweite Stielbogen 291 geht über in einen dritten Stielbogen 292, der als gaumenseitiger Stielendbogen ausgebildet ist. Der dritte Stielbogen 292 folgt dem weichen Gaumen eines Mundes. Ein vierter Stielbogen 295 liegt dem ersten Stielbogen 290 gegenüber und kann auch als zungenseitiger Stielansatzbogen bezeichnet werden. Der vierte Stielbogen 295 dient der Anlage der unteren Schneidezähne eines nicht dargestellten Mundes. Ein fünfter Stielbogen 296 schließt an den vierten Stielbogen 295 an und bildet einen zungenseitigen Stielmittenbogen. Der fünfte Stielbogen 296 dient zur Anlage an den anterodorsalen Bereich eines Zungenrückens. Der fünfte Stielbogen 296 geht über in einen sechsten Stielbogen 297, der auch als zungenseitiger Stielendbogen bezeichnet werden kann. Der sechste Stielbogen 297 dient zur Anlage an den posterodorsalen Bereich des Zungenrückens. Der gaumenseitige Stielendbogen 292 und der zungenseitige Stielendbogen 297 sind über einen siebten Stielbogen 299 miteinander verbunden, der das mundhöhlenseitige Ende des Mundhöhlenkörpers 202 bildet. Die Längserstreckung 203 des Mundhöhlenkörpers 202 geht bis zu dem siebten Stielbogen 299. Die Längserstreckung 203 wird von einer Längserstreckung 266 des kastenförmigen Körpers 260 fortgesetzt, die auch als außermundige Längserstreckung 266 bezeichnet werden kann.

Wie in Figur 3 zu sehen ist, ergeben die Längserstreckungen 203, 266, also die Längserstreckung 203 des Mundhöhlenkörpers 202 und die Längserstreckung 266 des kastenförmigen Körpers 260, zusammen eine Gesamtlänge des Mundsteckstücks 201. Der kastenförmige Körper 260 ist in den Mundschutz 268 eingesetzt. Seitlich an dem Gehäuse 262 des kastenförmigen Körpers 260 befindet sich ein Einschalttaster 301, der gegen eine rückstellende Tasterfeder 303 betätigbar ist. Der Einschalttaster 301 und die Tasterfeder 303 sind Teile des Gehäuses 262. Einschalttaster 301 und Tasterfeder 303 sind einstückig aus dem Gehäuse 262 heraus ausgeformt.

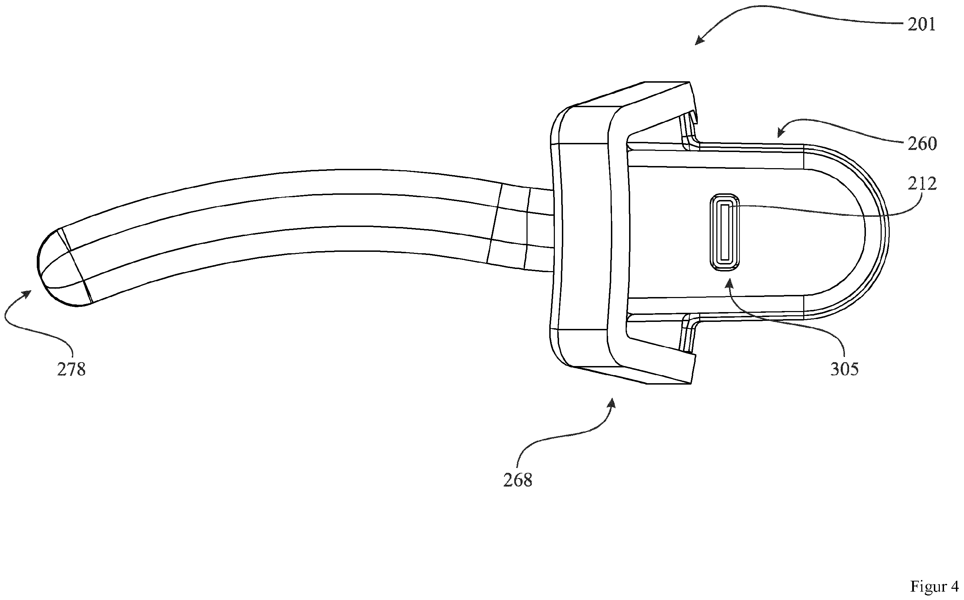

Figur 4 zeigt eine Seite des Mundsteckstücks 201, die der in Figur 3 dargestellten Seite gegenüberliegt. Der kastenartige Körper 260 bildet eine Fortsetzung des bogenförmigen Stiels 278. An dem kastenförmigen Körper 260 ist eine USB-Buchse 305 vorhanden. Die USB-Buchse 305 dient als elektrischer Anschluss 212 zur Versorgung des Mundsteckstücks mit Energie bzw. mit elektrischem Strom. Der bogenförmige Stiel 278 wird durch den Mundschutz 268 gegenüber dem elektrischen Anschluss 212 abgeschirmt. Die USB-Buchse 306 ist für eine Kleinspannung und einen Niedrigstrom ausgelegt, sodass selbst bei einer Berührung der Buchse 305 mit (nicht dargestellten) Lippen keine bemerkenswerten elektrischen Schläge auftreten können.

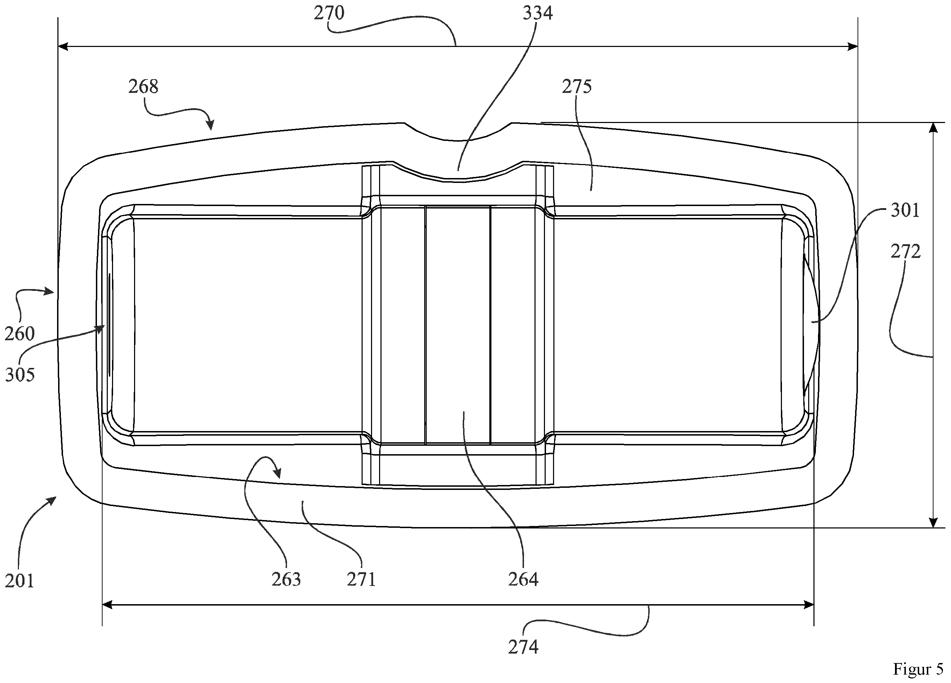

In Figur 5 ist eine schematisch dargestellte Draufsicht auf den kastenförmigen Körper 260 des Mundsteckstücks 201 gezeigt. Der Mundschutz 268 ist mit einer Anschlusslippe 271 auf eine Anschlusskante 263 des kastenförmigen Körpers 260 aufgesteckt und wird durch eine Kippnase 334, die in den Mundschutz 268 eingearbeitet ist, in Position gehalten. An dem kastenartigen Körper 260 befinden sich an dessen Schmalseiten, an jeweils einander gegenüberliegenden Seiten zum einen die USB-Buchse 305 und zum anderen der Einschalttaster 301. Mittig zwischen USB-Buchse 305 und Einschalttaste 301 ist der Griff 264 an dem kastenförmigen Körper 260 angeordnet, der zur besseren Handhabung eine Oberflächengriffstruktur (nicht eingezeichnet) aufweist, wobei die Oberflächengriffstruktur durch eine Aufrauhung realisiert ist. In unmittelbarer Nähe zur Anschlusskante 263 bzw. zur Kippnase 334 sind in dem Mundschutz 268 Durchlüftungsöffnungen, wie die Durchlüftungsöffnung 275, eingearbeitet, die mit einem Keimfilter (von außen nicht zu sehen) ausgestattet sind. Die Durchlüftungsöffnungen 275 erleichtern einer das Mundsteckstück 201 tragendenden Person das Atmen durch den Mund, obwohl der Mund durch den Mundschutz 268 gegenüber der Umwelt verschlossen ist. Der Mundschutz 268 weist eine Breite 270 auf, die größer ist als dessen Höhe 272. Der kastenartige Körper 260 weist eine Breite 274 auf, die kleiner ist als die Breite 270 des Mundschutzes 268. Damit ist das Mundsteckstück 201 besonders günstig zwischen Nase und Kinn im Gesicht einer Person zu positionieren und kann trotzdem angenehm getragen werden.

In den Figuren Figur 6, Figur 7, Figur 8 und Figur 9 sind verschiedene Ansichten einer zweiten Ausführungsform eines Mundsteckstücks 401 bzw. einzelner Teile des Mundsteckstücks 401 wie ein Gehäuse 462 gezeigt.

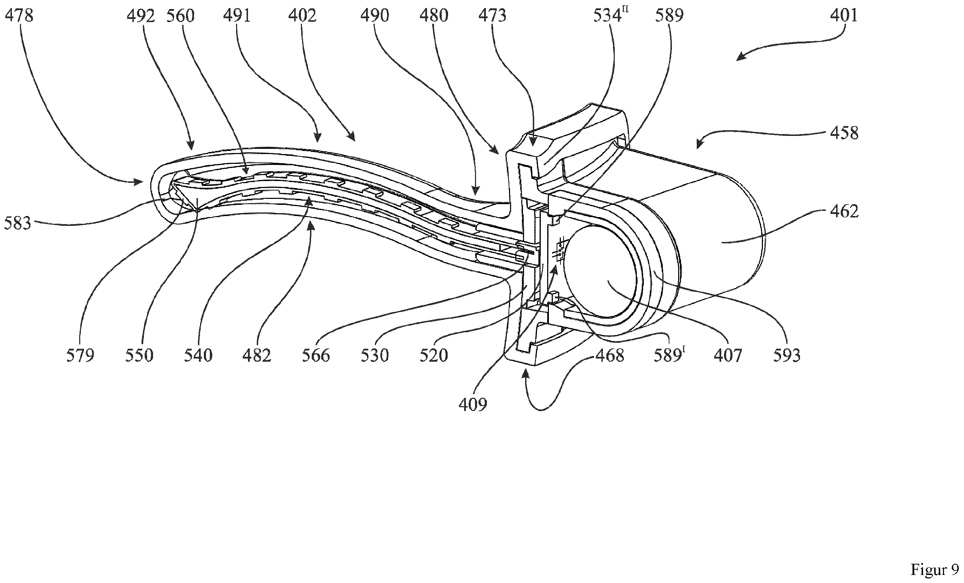

In der Figur 6 ist eine perspektivische Explosionsansicht des Mundsteckstücks 401 zu sehen. Hierdurch sind auch viele im Inneren 540 angeordnete Teile wie die LEDs 571, 572, 573, 577, 578, 579 zu sehen, die in einem zusammengebauten Zustand des Mundsteckstücks 401 (s. die Querschnittsdarstellung nach Figur 9) durch das Gehäuse 462 und den Mundhöhlenkörper 402 eingeschlossen sind. Das Gehäuse 462 ist als blockartiger Körper 458 ausgebildet, der einen Einschalttaster 501 und einen Griff 464 aufweist. Der Griff 464 ist bündig mit dem Gehäuse 462 auf dieses aufschiebbar bzw. aufklippbar, wobei eine durch den Griff 464 gebildete, somit an dem Gehäuse 462 vorhandene Betriebszustandsanzeige 593 auf diese Weise abgedeckt ist.

Ein Licht, insbesondere farbiges Licht, der Betriebszustandsanzeige 593 wird über eine Reflektorfläche 465 an dem Griff 464 nach außen hin abgestrahlt bzw. in ihrer Wirkung verstärkt. Diese als Spiegelfläche wirkende Reflektorfläche kann in einer alternativen (bildlich nicht dargestellten) Ausführungsvariante auch als, vorzugsweise weißliche, Lichtstreufläche ausgebildet sein.

Im Inneren des in Figur 6 gezeigten blockartigen Körpers 458 ist ein Batteriefach 510 vorhanden, in das ein handelsüblicher Akkumulator 407 passt (z. B. des Typs "18650" oder des Typs "32650" oder des Typs "32700"). Der Akkumulator 407 ist mit einer Steuerplatine 520 elektrisch verbunden, die ebenfalls in das Batteriefach 510 gehört. Auf der Steuerplatine 520 befindet sich seitlich ein Schalter 504, genauer gesagt, ein Mikroschalter, der durch den Einschalttaster 501 betätigbar ist. Wobei über einen Hebel (nicht dargestellt) auch ein Versatz zwischen Einschalttaster 501 und Schalter 504 ausgeglichen werden kann.

Die Steuerplatine 520 trägt zwei Betriebszustands-LEDs, wie die Betriebszustands-LED 589. Nachdem in einem betriebsbereiten Zustand des Mundsteckstücks 401 der Einschalttaster 501 betätigt wurde, wird von der Betriebszustands-LED 589 Licht in einen Lichtkanal 591 des Batteriefachs 510 an dem Akkumulator 407 vorbei auf das transluzente Fenster 593 der Betriebszustandsanzeige gestrahlt. Durch das Fenster 593 tritt Licht aus, wenn der Betriebszustand eingenommen ist, und wird von einer benachbarten Person unmittelbar erkannt oder über die Reflektorfläche 465 von der das Mundsteckstücks 401 tragenden Person gesehen.

Wie des Weiteren in Figur 6 zu sehen ist, ist über einen Steckverbinder 558 die Lichtquelle 406 an die Steuerplatine 520 elektrisch anschließbar. Der Steckverbinder 558 ermöglicht es, das Mundsteckstück 401 in zwei Teile auseinanderzunehmen, von denen ein erstes Teil der blockartige Körper 458 ist und ein zweites Teil der Mundhöhlenkörper 402 ist. Die Verbindung wird über Kippnasen, wie die Kippnase 534, zusammengehalten. Von dem blockartigen Körper 458 kann der Mundhöhlenkörper 402 abgenommen und durch einen anderen Mundhöhlenkörper, z. B. durch einen Mundhöhlenkörper mit einer größeren Länge oder durch einen Mundhöhlenkörper mit einem breiteren transluzenten Körper als dem transluzenten Körper 476, ersetzt werden. Der Steckverbinder 558 führt durch eine Abschlussplatte 530 hindurch zu einem Anschraubverbinder 556, auf dessen LED-Träger 550 die Lichtquelle 406 angeordnet ist. Die Lichtquelle 406 umfasst einen oberen LED-Strang 562 und einen unteren LED-Strang 564, die jeweils Licht in einander entgegengesetzte Richtungen abstrahlen. Die Lichtquelle 406 ist über elektrische Kontakte 566 zur Stromversorgung mit dem Steckverbinder 558 verbunden, wenn das Mundsteckstück 401 zusammengebaut ist (s. auch die Kontakte 566 in Figur 9). Durch Auflage des Mundschutzes 468 auf der Dichtfläche 532 ist das Innere 540 des blockartigen Körpers 458 flüssigkeitsdicht abgeschlossen.

Auf dem LED-Träger 550 ist ein Temperatursensor 581 vorhanden, der gemessene Temperaturwerte in Gestalt von Messdaten auf in dem LED-Träger 550 verlaufenden Datenleitungen zur Steuerplatine 520 sendet, in dem die Signale über den Steckverbinder 558 hinweg geleitet werden. Der Temperatursensor 581 ist Teil einer Temperaturüberwachung. Der Temperatursensor 581 kann sowohl eine Umgebungstemperatur, z. B. eine Fiebertemperatur im Mund eines Menschen, als auch eine LED-Betriebstemperatur erfassen. Für eine besonders zuverlässige Fiebermessung ist der Temperatursensor 581 mit einem Fühler auszustatten, der an einer Unterseite des Mundhöhlenkörpers hinaus zur Zunge reichen kann.

Die Lichtquelle 406 in Figur 6 umfasst eine größere Anzahl LEDs 571, 572, 573, 577, 578, 579, genauer gesagt 17 LEDs, die zusammen auf einem LED-Band 560 (auch als LED-Stripe bezeichnet) angeordnet sind. Die Stromversorgung dieser LEDs 571, 572, 573, 577, 578, 579 erfolgt aus dem Akkumulator 407 über die Steuerplatine 520, den Steckverbinder 558 und über LED-Stromversorgungskontakte 566 hinweg. Eine erste nach unten gerichtete Blaulicht-LED 572 dient der Bestrahlung des sublaminalen Bereichs im Mundraum. Eine auf dem LED-Band in einem endnahen Bereich angeordnete Gruppe von zwei UV-C-Licht-LEDs 577, 578 dient der Bestrahlung eines tieferliegenden Bereichs der Mundhöhle. Am Ende des LED-Trägers 550 ist eine Laser-LED 579 angeordnet, deren Licht über eine Mikrolinsenoptik 583 des Mundhöhlenkörpers 402 in Bereichen des Rachens verteilt wird (siehe auch die anatomischen Gegebenheiten in Figur 10). Zwischen den UV-C-Licht-LEDs 577, 578 und einer ersten LED 571 bzw. einer dieser LED 571 gegenüberliegenden LED 572 sind weitere Blaulicht-LEDs, wie die Blaulicht-LED 573 angeordnet, deren Licht in einem Betriebszustand des Mundsteckstücks 401 durch den transluzenten Körper 476 austritt. In einem solchen Betriebszustand ist der Mundhöhlenkörper 402 bis zu dessen Anlagefläche 404, die sich an dem Mundschutz 468 befindet, in eine Mundhöhle eingesteckt (vgl. Mundhöhle 20 in Figur 1 bzw. Mundhöhle 20^{I} in Figur 10).

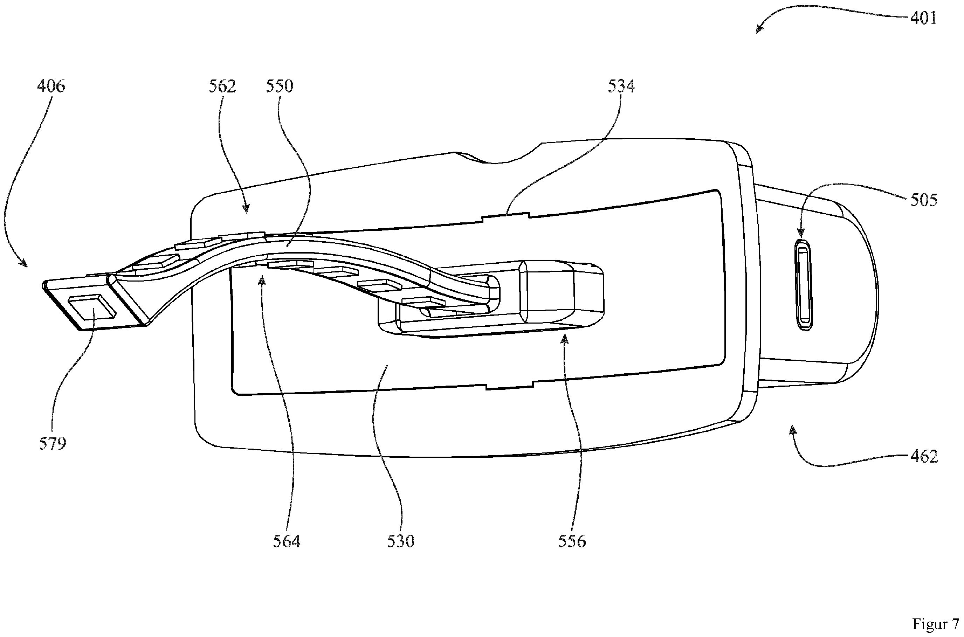

Figur 7 stellt ein teilweise zusammengebautes Mundsteckstück 401 dar, dessen Einzelteile aus Figur 6 bekannt sind. Das Gehäuse 462 weist eine USB-Buchse 505 auf. In das Gehäuse 462 ist mit Hilfe von Kippnasen, wie der Kippnase 534, die Abschlussplatte 530 eingesetzt. An der Abschlussplatte 530 ist der Schraubverbinder 556 rücklings angeschraubt, der den LED-Träger 550 trägt. Auf dem LED-Träger 550 sind der obere LED-Strang 562 und der untere LED-Strang 564 montiert, die der geschwungenen Form des LED-Trägers 550 folgen. Die Laser-LED 579 sitzt am Ende des Trägers 550 und erlaubt es, ausgehend von dem LED-Träger 550 entfernte und tiefergelegene Bereiche des Rachens mit Licht zu bescheinen. Laserlicht lässt sich besonders gut durch eine vor die Laser-LED 579 gesetzte Optik in eine gewünschte Richtung führen und dabei aufweiten (siehe z. B. die in Figur 6 gezeigte Mikrolinsenoptik 583). Um den anatomischen Gegebenheiten (s. Figur 10) gerecht zu werden, führt eine Vorzugsstrahlrichtung in einem Winkel von dem Träger 550 bzw. der Abschlussplatte 530 weg. Die beiden LED-Stränge 562, 564 und die Laser-LED 579 bilden zusammen mit dem Träger 550, der eine Orientierung der darauf angeordneten LEDs 562, 564, 469 vorgibt, die Lichtquelle 406.

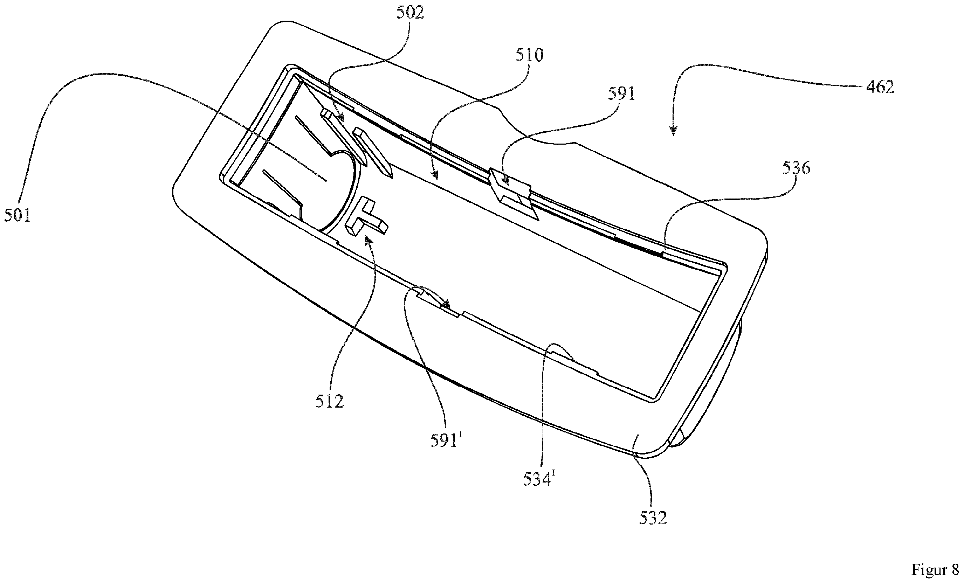

In Figur 8 ist das Gehäuse 462, das aus Figur 6 und Figur 7 bereits bekannt ist, mit einer Blickfreigabe in das Batteriefach 510 gezeigt. Das Batteriefach 510 wird durch das Gehäuse 462 gebildet. Das Gehäuse 462 ist von der Dichtfläche 532 umschlossen. An der Dichtfläche 532 befindet sich eine Dichtkante 536 mit mehreren von Kippnasen, wie z. B. die Kippnase 534^{I}, die dazu dienen, eine Abschlussplatte, wie die in Figur 7 gezeigte Abschlussplatte 530, zu halten und das Batteriefach 510 damit zu verschließen. In dem Gehäuse 462 bzw. in dessen Gehäusewand sind zwei Lichtkanäle 591, 591^{I} eingearbeitet, die der Betriebszustandsanzeige dienen. Das Gehäuse 462 weist den Einschalttaster 501 auf, neben dem sich eine Hebelführung 502 und ein Tasteranschlag 512 befinden.

Eine Querschnittsdarstellung des Mundsteckstücks 401, das bereits aus den Figuren Figur 6 bis Figur 8 bekannt ist, ist in Figur 9 in perspektivischer Ansicht gezeigt. In dem Gehäuse 462, das als blockartiger Körper 458 realisiert ist, sitzt der Akkumulator 407 und die Steuerplatine 520. Die Steuerplatine 520 ist durch die Abschlussplatte 530 abgestützt ist. Die Steuerplatine 520 trägt auf jeder ihrer Langseiten, ungefähr im mittleren Bereich, jeweils eine Seiten-LED 589, 589^{I} zur Anzeige eines Betriebszustands des Mundsteckstücks 401 mittels blauem Licht, das durch das transluzente Fenster 593 nach außen fällt. Die Abschlussplatte 530 bildet einen Schutz für die Steuerplatine 520 gegen Feuchtigkeit bzw. erhöhte Kondensatsättigung in der Luft und auch gegen mechanische Angriffe. Der Mundschutz 468 weist einen Überwurf auf, der über einen Rand des blockartigen Körpers 458 darüber gestülpt werden kann. Mit Hilfe einer Klippnase 534" wird der Mundschutz 468 mit dem blockartigen Körper 458 zusammengehalten. Die Klippnase 534^{II} ist an einer Dehnungsmulde 473 manuell rückziehbar. Der Mundschutz 468 ist als ein Lippenanschlusskörper 480 ausgeformt. Von dem Lippenanschlusskörper 480 weg erstreckt sich der Mundhöhlenkörper 402 als bogenförmiger Stiel 478, der mehrere Bögen aufweist, wie einen ersten Bogen 490, einen zweiten Bogen 491 und einen dritten Bogen 492. Im Inneren 540 des Mundhöhlenkörpers 402 erstrecken sich der LED-Träger 550 mit dem LED-Band 560, wobei der LED-Träger 550 dem Verlauf der Stielbögen 490, 491, 492 folgt. Licht, das von der Laser-LED 579 ausgeht, fällt auf die Mikrolinsenoptik 583, durch die das Licht auf tieferliegende Bereiche des Rachens (vgl. Figur 10) gerichtet wird. Das LED-Band 560 wird über die LED-Stromversorgungskontakte 566 mit für die LEDs geeigneter Versorgungsspannung versorgt. Alle elektronischen Komponenten des Mundsteckstücks 401 sind von einer Kunststoffhülle 482 eingeschlossen, damit kein Strom über den Körper einer das Mundsteckstück 401 nutzenden Person fließen kann. Die Kunststoffhülle 482 ist eine elektrisch isolierende Kunststoffhülle, die aus einem körperverträglichen, formadaptiven Kunststoff, wie einem Latex oder einem Silikon, besteht.

Die Steuerplatine 520 kann mehrere Steuerfunktionen umsetzen, die von einem Steuerprozessor geleistet werden. Der Steuerprozessor arbeitet mit einem auf der Steuerplatine 520 angeordneten Datenspeicher und mit einer auf der Steuerplatine befindlichen USB-Schnittstelle (nicht dargestellt) zusammen. Die Steuerplatine 520 überwacht den Ladezustand des Akkumulators 407 sowie Lebensdaten bzw. Gesundheitsdaten des Akkumulators 407. Wenn ein Ladestromkabel angeschlossen ist (siehe die in Figur 7 gezeigte USB-Buchse 505), regelt die Steuerplatine 520 den Ladestrom. Die Steuerplatine 520 kontrolliert die Zufuhr von elektrischem Strom zu den LEDs 571, 572, 573, 577, 578, 579 des Mundsteckstücks 401.

Werden die Figuren Figur 6, Figur 7, Figur 8 und Figur 9 nun gemeinsam betrachtet, so ist zu sehen, wie die Gruppen von LEDs, wie eine Gruppe von Blaulicht-LEDs 571, 572, 573 und eine Gruppe von UV-C-LEDs 577, 578, durch die Steuerplatine 520 unabhängig voneinander mit Strom versorgbar sind. Die Steuerplatine 520 umfasst insbesondere eine Zeitsteuerung 409. Mit der Zeitsteuerung 409 können Blaulicht-LEDs 571, 572, 573 sowie UV-C-LEDs 577, 578 bzw. die Laser-LED 579 jeweils für eine unterschiedliche Zeitdauer eingeschaltet werden. Über die Zeitsteuerung 409 kann eine Zeitsperre gesetzt werden. Die Zeitsperre verhindert, dass an eine erste Bestrahlungsdauer eine zweite Bestrahlungsdauer ohne Einhaltung einer vorgegebenen Wartezeit angeschlossen wird. Die Steuerplatine 520 empfängt aktuelle Temperaturmessdaten von dem Temperatursensor 581. Wenn ein empfangener Temperaturmesswert eine in dem Steuerprozessor gesetzte Maximaltemperatur überschreitet, wird der Betrieb des Mundsteckstücks 401 durch die Zeitsteuerung 409 unterbrochen. Die Steuerplatine 520 bzw. der darauf enthaltene Steuerprozessor ist über den USB-Anschluss 505 mit den erforderlichen Daten bzw. Betriebsparametern programmierbar. Beispielsweise kann ein Behandlungsplan in den Steuerprozessor geladen werden, durch den unterschiedliche Schaltdauern für die Zeitsteuerung 409 vorgegeben werden. Die Steuerplatine 520 erfasst anhand einer Kennung, welche Art und/oder welche Größe eines Mundhöhlenkörpers, wie der Mundhöhlenkörper 402, angeschlossen ist. Anschlussdaten sowie Betriebsdaten, insbesondere Temperaturdaten, oder auch Akkumulatordaten können über den USB-Anschluss 505, z. B. im Abfragegerät, wie ein Laptop, ausgelesen werden. Auf dem mobilen Abfragegerät (nicht dargestellt) kann eine Software installiert sein, die es erlaubt, einen Therapieplan zu erstellen und auf das Mundsteckstück 401 zu übertragen. Damit lässt sich der Betrieb des Mundsteckstücks 401 besonders gut überwachen. Insbesondere ist ein Therapieplan auf eine aktuell gemessene Körpertemperatur abstimmbar.

Figur 10 skizziert die anatomischen Gegebenheiten im Bereich einer Mundhöhle 20^{I} einer Person, wobei die Person in ihrer Mundhöhle 20^{I} ein Mundsteckstücks 601 trägt. Das in Figur 10 gezeigte Mundsteckstück 601 ist eine weitere Variante zu dem Mundsteckstück 1 nach Figur 1, das, wie das Mundsteckstück 1 in Figur 1 es auch hat, einen knopfartigen Körper 611 aufweist. An dem knopfartigen Körper 611 des Mundsteckstücks 601 befindet sich ein Einschalttaster 701. Nach Betätigung des Einschalttasters 701 wird eine weitere Übernahme eines elektrischen Schaltimpulses von dem Einschalttaster 701 während der Dauer eines voreingestellten Zeitintervalls, d. h. während der Dauer eines zweiten Zeitintervalls, ignoriert, gesperrt oder verriegelt.

Durch einen transluzenten Körper 676 des Mundsteckstücks 601 hindurch wird für die Dauer eines ersten Zeitintervalls, das kürzer als das zweite Zeitintervall ist, Licht abgestrahlt.

Wie anhand von Figur 10 zu sehen ist, liegt eine Oberlippe 23^{I} mit dem Amorbogen 23^{II} an einer Auflage 605 eines Mundschutzes 668 des Mundsteckstücks 601 an. Der Mundschutz 668 verhindert somit, dass ein restlicher Teil des Mundsteckstücks 601, der außerhalb des Mundes verbleiben soll, an den Lippen 23^{I}, 24^{I} bzw. an den Schneidezähnen 21^{I}, 22^{I} vorbei in den Rachen 42 hinein gelangen kann. In Figur 10 sind der Oberkiefer 38 und der Unterkiefer 40 leicht geöffnet dargestellt. Bei einem geöffneten Mund durch Beabstandung von Oberkiefer 38 und Unterkiefer 40 wird die Einführung des Mundsteckstücks 601 erleichtert. Durch Schließen von Unterkiefer 40 und Oberkiefer 38 gelangen die Schneidezähne 21^{I}, 22^{I} in eine Position, in der die Schneidezähne 21^{I}, 22^{I} auf dem transluzenten Körper 676 aufliegen. Ein anterodorsaler Bereich 32 und ein posterodorsaler Bereich 34 des Rückens der Zunge 25^{I} können an dem transluzenten Körper 676 zur Anlage kommen. Zwischen den unteren Schneidezähnen 22' und der Zunge 25^{I} befindet sich ein sublaminaler Bereich 36, der durch den transluzenten Körper 676 hindurch mit blauem Licht bestrahlbar ist. Von dem Mundhöhlenkörper 602 wird oberseitig durch den transluzenten Körper 676 hindurch der harte Gaumen 26^{I}, der weiche Gaumen 28 und das Gaumenzäpfen 30 bestrahlt. Außerdem wird von dem Mundhöhlenkörper 602 Licht in den Bereich des Rachens 42, insbesondere in Richtung des Kehldeckels 48 bzw. der Luftröhre 50 abgegeben. Während der Bestrahlung der Mundhöhle 20^{I} mit Licht aus dem Mundhöhlenkörper 602 kann die Person durch die Nasenhöhle 44 über den Nasenrachen 46 Luft, insbesondere durch Nasenhärchen gefiltert, einatmen. Ein Eintritt von Influenza-Viren in den Rachenbereich 42 ist erschwert, weil der Mundhöhlenkörper 602 den Eintritt behindert.

In einer nicht dargestellten Weiterbildung kann der Mundhöhlenkörper an seinem Ende zweigeteilt ausgeführt sein, um das in Figur 10 zu sehende Gaumenzäpfchen 30 seitlich zu passieren und besonders weit in den Rachen 42 des das Mundsteckstück nutzenden Menschen hineinzuleuchten.

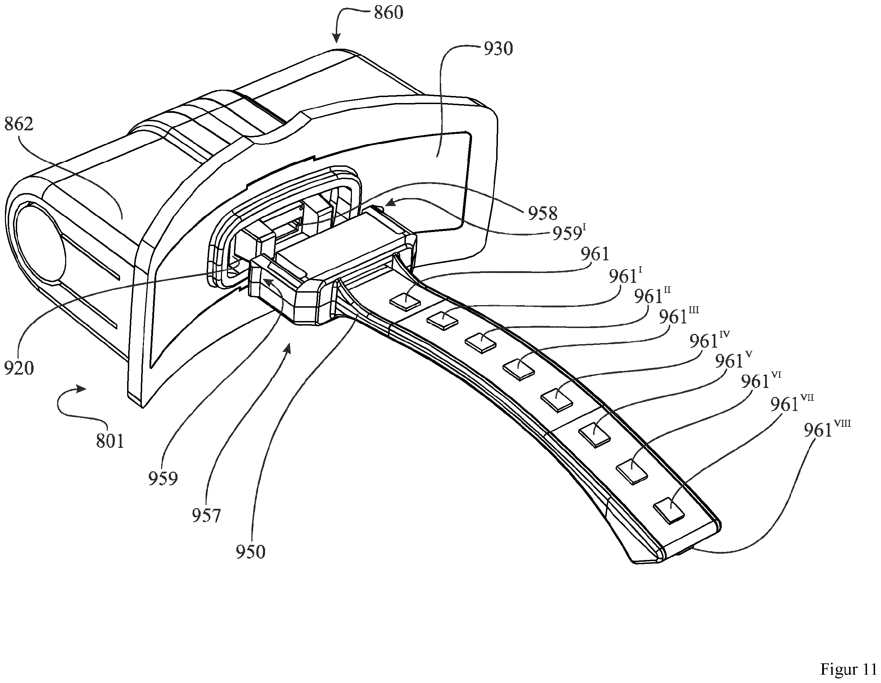

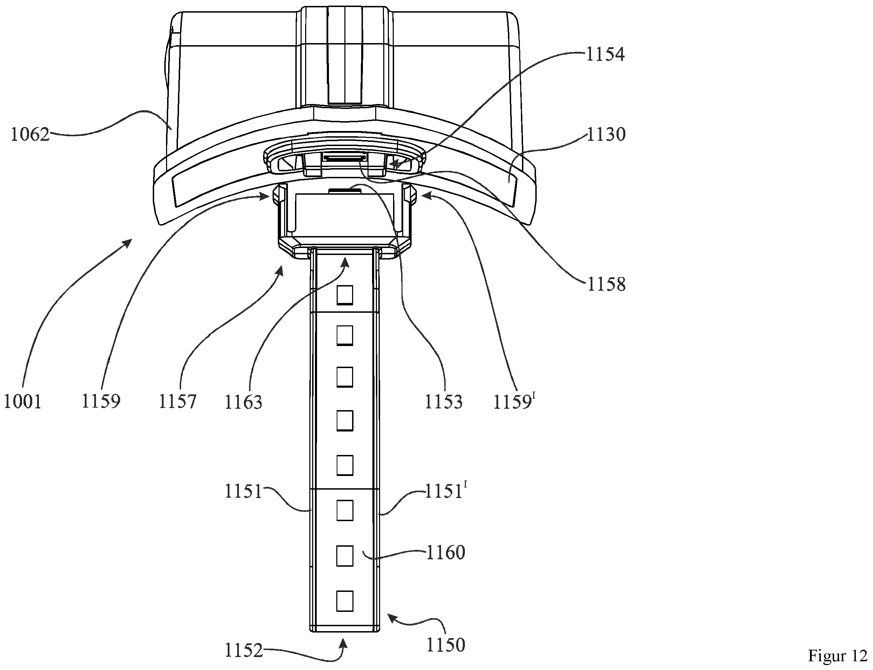

Die in den Figuren 11 und 12 gezeigten beiden Ausgestaltungsformen eines Mundsteckstücks 801, 1001 sind ohne eine abschließende Hülle, z. B. ohne die - in Figur 2 gezeigteabschließende, die Innereien schützende Hülle 282, dargestellt, um die Anordnung der LEDs 961, 961^{I}, 961^{II}, 961^{III}, 961^{IV}, 961^{V}, 961^{VI}, 961^{VII}, 961^{VIII}, die durch die Hülle normalerweise geschützt sind, besser sehen zu können. Die in Figur 2 gezeigte Hülle 282 übernimmt in einer günstigen Ausgestaltung nicht nur die Funktion einer Schutzhülle, z. B. gegen Bisse, sondern auch die Funktion eines Diffusors, um das aus den LEDs, wie den LEDs 961, 961^{I}, 961", 961^{III}, 961^{IV}, 961^{V}, 961^{VI}, 961^{VII}, 961^{VIII}, austretende, punktuelle Licht gleichmäßiger zu streuen.

Die in Figur 11 gezeigten LEDs 961, 961^{I}, 961", 961^{III}, 961^{IV}, 961^{V}, 961^{VI}, 961^{VII}, 961^{VIII} sind auf einem LED-Träger 950 befestigt. Der LED-Träger 950 unterstützt die flexible Platine, die die LEDs 961, 961^{I}, 961^{II}, 961^{III}, 961^{IV}, 961^{V}, 961^{VI}, 961^{VII}, 961^{VIII} über Leiterbahnen mit Strom versorgt. Der LED-Träger 950 mündet in einen Clipverbinder 957. An dem Clipverbinder 957 sind elektrische Kontakte (in der gezeigten Perspektive nicht zu sehen) vorhanden, die in einen Steckverbinder 958 einführbar sind. Der Steckverbinder 958 ist auf der Steuerplatte 920 angebracht; er ist Teil der Steuerplatine 920, die quer zum LED-Träger 950 im Gehäuse 862 liegt. Über den Steckverbinder 958 kann Strom zu allen LEDs 961, 961^{I}, 961", 961^{III}, 961^{IV}, 961^{V}, 961^{VI}, 961^{VII}, 961^{VIII} fließen, wenn seitliche Clips 959, 959^{I} an dem Clipverbinder 957 den LED-Träger 950 mit einem kastenartigen Körper 860 zusammenhalten. Die zwei Clips 959, 959^{I} sind an einander gegenüberliegenden Seiten des Clipverbinders 957 so angeordnet, dass sie an einer Abschlussplatte 930 eines Gehäuses 862 einrasten können, wobei ein Halt durch zurückfederndes Verspreizen der zwei Clips 959, 959^{I} entsteht. Ein Benutzer (nicht dargestellt), der mit seinen Fingern einer Hand an den zwei Clips 959, 959^{I} des Clipverbinders 957 angreift, kann die zwei Clips 959, 959^{I} aufeinander zu zusammendrücken und den Clipverbinder 957 von der Abschlussplatte 930 abziehen.

Gleichartig zu dem in Figur 11 gezeigten Stecksystem können bei einer Ausführungsform in Übereinstimmung mit Figur 12 Steckkontakte 1153 an einem Clipverbinder 1157 entlang eines Kontaktführungspaars 1154 in den Steckverbinder 1158 eingeschoben werden. Der Clipverbinder 1157 wird dann durch Einrasten der Clips 1159, 1159^{I} mit der Endplatte bzw. Abschlussplatte 1130 gekoppelt. Die Endplatte bzw. Abschlussplatte 1130 ist flüssigkeitsdicht in ein Gehäuse 1062 des Mundsteckstücks 1001 eingeklebt. In den Clipverbinder 1157 ist ein LED-Träger 1150 eingesetzt. Der LED-Träger 1150 weist einen ersten Seitenschutz 1151 und einen zweiten Seitenschutz 1151^{I} auf, die eine auf dem LED-Träger 1150 angeordnete LED-Platine 1160, die auch als LED-Band 1160 bezeichnet werden kann, flankieren. Der Seitenschutz 1151, 1151^{I} bietet eine Fingergrifffläche. Außerdem bewahrt der Seitenschutz 1151, 1151^{I} die vertieft angeordnete LED-Platine 1160 mit deren auf ihr etwa äquidistant in einer Reihe angeordneten LEDs vor Verunreinigungen durch Fingerabdrücke. Eine Trägerrundung 1152 an dem LED-Träger 1150, d. h. an dessen Ende, ist als Endrundung zylindermantelabschnittartig ausgebildet.

Wird das Mundsteckstück 1001 aus seinen Einzelteilen zusammengebaut, so wird die LED-Platine 1160 um die Trägerrundung 1152 herum eng und knickfrei an den LED-Träger 1150 angelegt. Es kann also gesagt werden, dass die LED-Platine 1160 durch die Trägerrundung 1152 einen Wendebereich in ihrer Längserstreckung hat. Die LED-Platine 1160 folgt ihrer Trägerrundung 1152. Anschließend werden der Clipverbinder 1157 auf ein der Trägerrundung 1152 gegenüberliegendes Ende des LED-Trägers aufgesetzt und die Steckkontakte 1153 mit der LED-Platine 1160 verbunden. Zwei Endbereiche der einstrangigen LED-Platine 1160, wie der Platinenendbereich 1163, führen somit in den Clipverbinder 1157 hinein und somit zu den Steckkontakten 1153.

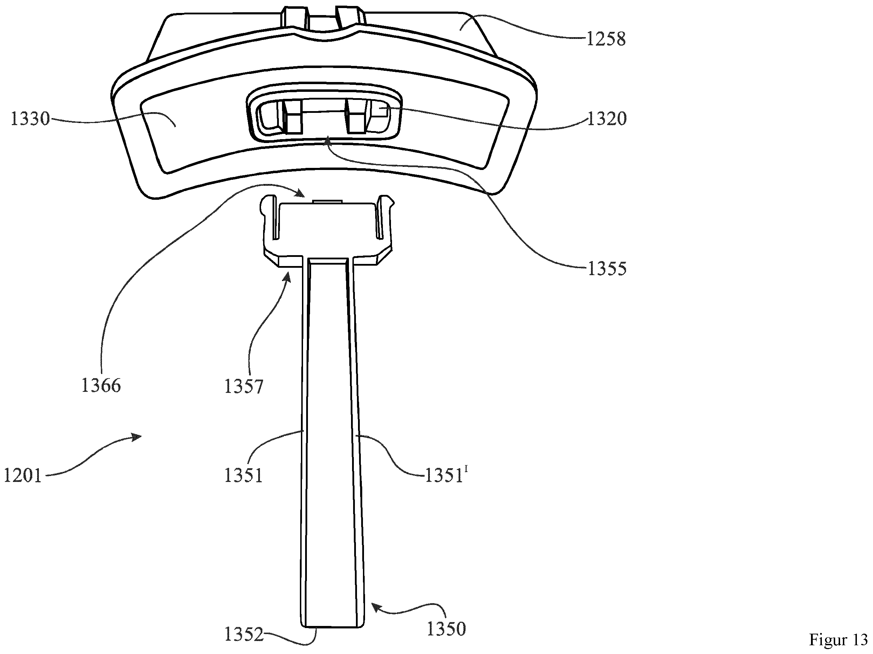

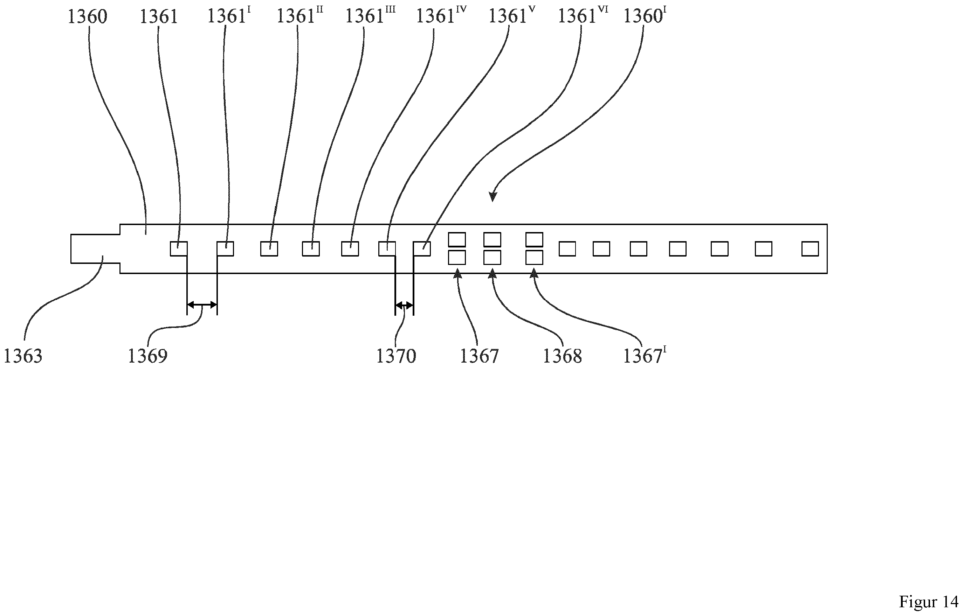

Die Figuren Figur 13 und Figur 14 stellen ein weiteres Ausführungsbeispiel für ein Mundsteckstücke vor, genauer anhand des Mundsteckstücks 1201. Fig. 13 und Fig. 14 zeigen Komponenten eines Mundsteckstücks 1201, wobei zu einer besseren Einsehbarkeit innenliegender Komponenten ein transluzenter Körper, wie der in Fig. 6 gezeigte transluzente Körper 476, weggelassen bzw. abgezogen wurde. Der transluzente Körper kann zur seiner Sterilisation von einem blockartigen Körper 1258 des Mundsteckstücks 1201 abgenommen werden. Das Mundsteckstück 1201 umfasst einen LED-Träger 1350, der in einer Trägerrundung 1352 endet. Ein erster Seitenschutz 1351 und ein zweiter Seitenschutz 1351^{I}, die sich an dem LED-Träger 1350 erstrecken, bilden eine Begrenzung einer rinnenartigen Vertiefung des LED-Trägers 1350. Zwischen dem ersten Seitenschutz 1351 und dem zweiten Seitenschutz 1351^{I} ist eine in Fig. 14 gezeigte LED-Platine 1360 einlegbar. Der erste Seitenschutz 1351 und der zweite Seitenschutz 1351^{I} erstrecken sich von einem Clipverbinder 1357 des LED-Trägers 1350 bis zu der Trägerrundung 1352. Die in Fig. 14 gezeigte LED-Platine 1360 wird während der Herstellung des Mundsteckstücks 1201 auf den LED-Träger 1350, der in Fig. 13 zu sehen ist, aufgeklebt. Ein Platinenendbereich 1363, der in Fig. 14 gezeigt ist, ist bis zu einem Kontaktfeld 1366 des Clipverbinders 1357 in dem LED-Träger 1350 einfädelbar. Das in Fig. 13 gezeigte Kontaktfeld 1366 kann in eine Einstecköffnung 1355 eines blockartigen Körpers 1258 eingeführt werden. Die Einstecköffnung 1355 ist in einer Abschlussplatte 1330 des blockartigen Körpers 1258 vorhanden. Die Abschlussplatte 1330 trägt in dem blockartigen Körper 1258 innenliegend zugleich alle Komponenten, wie eine Steuerplatine 1320, die von einem ebenfalls innenliegenden, und daher in Figur 13 nicht sichtbaren Akkumulator mit elektrischem Strom versorgt wird. In entsprechender Weise kann z. B. der LED-Träger 1350, dessen LED-Platine 1360 defekt ist, herausgenommen und ersetzt werden.

In einem Reinigungsverfahren kann ein transluzenter Körper, wie der in Fig. 6 gezeigte transluzente Körper 476, von Schmutz befreit werden. Für ca. 30 Sekunden kann der transluzente Körper in eine Desinfektionslösung, wie einen mehr als siebzigvolumenprozentigen Alkohol, ggf. mit einer Beimischung von Wasserstoffperoxid, eingetaucht werden. Alternativ kann der transluzente Körper in einer wässrigen Seifenlösung ausgekocht und anschließend mit gereinigtem Wasser gespült werden. Nach dem Trocknen kann der transluzente Körper wieder über den LED-Träger 1350 gezogen werden. Damit sind insbesondere Speichelreste von dem transluzenten Körper entfernbar.

Die LED-Platine 1360, die in Fig. 14 gezeigt ist, ist biegsam. Auf der LED-Platine 1360 befinden sich mehrere vereinzelte LEDs 1361, 1361^{I}, 1361^{II}, 1361^{III}, 1361^{IV}, 1361^{V}, 1361^{VI}. Auf die Reihe von Einzel-LEDs 1361, 1361^{I}, 1361^{II}, 1361^{III}, 1361^{IV}, 1361^{V}, 1361^{VI} folgen in einem Platinenmittenbereich 1360 drei LED-Paare 1367, 1368, 1367^{I}, denen auf der LED-Platine 1360 wieder eine Reihe von Einzel-LEDs folgt, ähnlich zu den links aufgereihten LEDs 1361 bis 1361^{VI}. Ein LED-Paar 1367, 1367^{I}, 1368 umfasst zwei einzelne LEDs, die in einem nächstmöglichen Abstand in einer Querrichtung zu der Einzel-LED-Reihung auf der LED-Platine 1360 angeordnet sind. Somit sind auf der LED-Platine 1360 genau 20 LEDs befestigt und über Leiterbahnen (nicht dargestellt) mit elektrischem Strom versorgbar. Auch sind aus Übersichtsgründen solche Bauteile wie Widerstände weggelassen worden. Ein mittleres LED-Paar 1368 der drei LED-Paare 1367, 1368, 1367^{I} wird auch als Rundungs-LED-Paar 1368 bezeichnet, weil dieses LED-Paar 1368 in einem Platinenmittenbereich 1360^{I} bei der Montage der LED-Platine 1360 auf dem LED-Träger 1350 (siehe Fig. 13) der Rundung 1352 nächst kommend angeordnet wird. Durch die Anordnung der Doppel-LEDs 1367, 1368, 1367^{I} in der Nähe der Trägerrundung 1352 ist von dem Bereich der Trägerrundung 1352 eine besonders hohe Lichtstärke abstrahlbar. Die LEDs der LED-Paare 1367, 1367^{I} sind näher zueinander angeordnet als die Einzel-LEDs 1361 bis 1361^{VI}. Zwischen einer ersten LED 1361 auf der LED-Platine 1360, die sich in einer Nähe zum Platinenendbereich 1363 befindet, und einer benachbarten, zweiten LED 1361^{I} gibt es einen Abstand 1369. Zwischen einer sechsten LED 1361^{V} und einer siebten LED 1361^{VI} existiert ein zweiter Abstand 1370, der kleiner ist als der erste Abstand 1369. Der erste Abstand beträgt 10 mm (Millimeter). Der zweite Abstand liegt bei 3 mm. Durch Wahl der Abstände 1369, 1370 lässt sich eine von den LEDs 1361 bis 1361^{VII} entlang des Mundsteckstücks 1201 bereitstellbare Leuchtstärke bzw. Lichtintensität vorgeben.

Beispielsweise sind auch solche Ausführungsformen möglich, bei denen der erste Abstand zwischen 5 mm und 12 mm groß ist und der zweite Abstand zwischen 3 mm und 6 mm groß ist. Anders gesagt ist es vorteilhaft, wenn in einer Mundhöhle (vgl. Mundhöhle 20^{I} in Fig. 10) im Bereich des Rachens 42) eine höhere Lichtintensität abgegeben wird als in einen Bereich der Schneidezähne 21^{I}, 22^{I}.

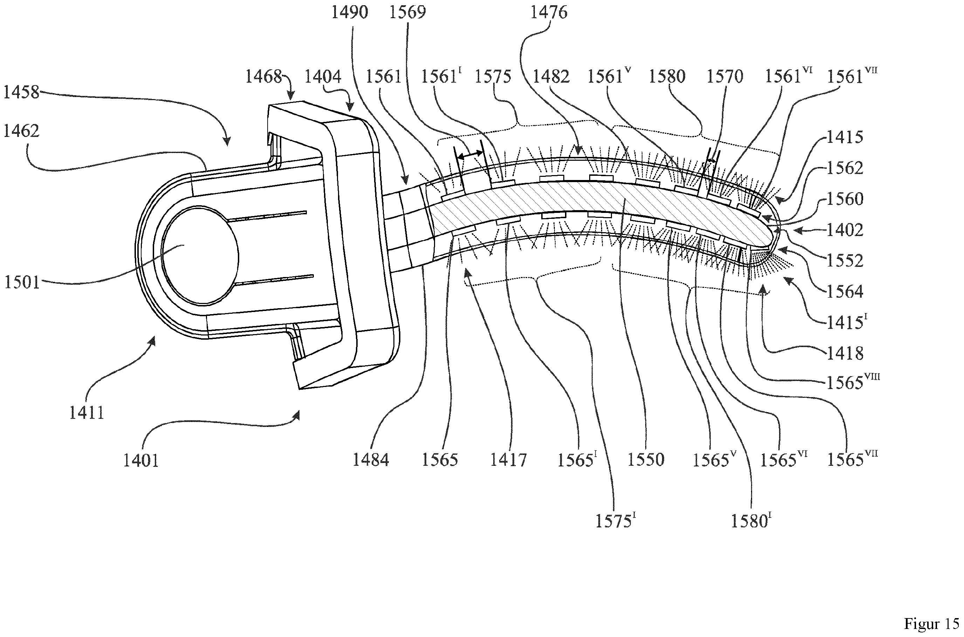

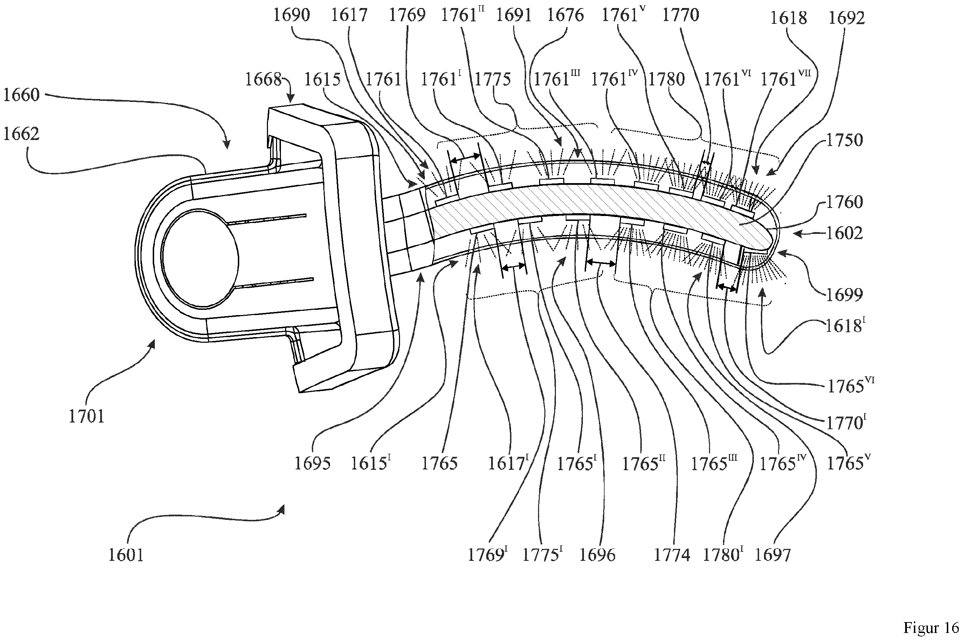

Figuren 15 und 16 zeigen zwei weitere Ausführungsbeispiele für Mundsteckstücke 1401, 1601.

Das Mundsteckstück 1401, das in Figur 15 gezeigt ist, hat einen ersten Bereich 1575, 1575^{I}, in dem der Mundhöhlenkörper 1402 mit einer ersten, d. h. niedrigeren Lichtdichte leuchtet und einen zweiten Bereich 1580, 1580^{I}, in dem der Mundhöhlenkörper 1402 mit einer zweiten, d. h. höheren Lichtdichte leuchtet. Die Helligkeit der LEDs 1561, 1561^{I}, 1561^{V}, 1561^{VI}, 1561^{VII}, 1565, 1565^{I}, 1565^{V}, 1565^{VI}, 1565^{VII}, 1565^{VIII} wird durch den elektrischen Strom bestimmt, der von der (nicht sichtbaren) Steuerplatine in dem blockartigen Körper 1458 für jede LED 1561, 1561^{I}, 1561^{V}, 1561^{VI}, 1561^{VII}, 1565, 1565^{I}, 1565^{V}, 1565^{VI}, 1565^{VII}, 1565^{VIII} individuell eingestellt wird.

Ein erster Teil des Mundsteckstücks 1401 wird durch den knopfartigen Körper 1411 gebildet. Es kann zudem gesagt werden, der Körper 1411 ist ein blockartiger Körper 1458, in dem eine Steuerungselektronik und ein elektrischer Speicher aufbewahrt sind. Dieses Gehäuse 1462, das durch den blockartigen Körper 1458 gebildet ist, ist als Griff gestaltet. Das Gehäuse 1462 hat an einer Seite einen Einschalttaster 1501.

Der zweite Teil des Mundsteckstücks 1401 wird durch den Mundhöhlenkörper 1402 gebildet. Zwischen dem Mundhöhlenkörper 1402 und dem Gehäuse 1462 gibt es eine trennende Wand, über die ein Mundschutz 1468 ausgebildet ist. Eine Seite des Mundschutzes 1468 ist als Anlagefläche 1404 gestaltet, sodass gegen die Anlagefläche 1404 Lippen und/oder Mundpartien eines Nutzers des Mundsteckstücks 1401 anliegen können. In dem Mundhöhlenkörper 1402 sind die LEDs, wie die LEDs 1561, 1561^{I}, 1561^{V}, 1561^{VI}, 1561^{VII}, 1565, 1565^{I}, 1565^{V}, 1565^{VI}, 1565^{VII}, 1565^{VIII}, angeordnet. Aus diesem Grund ist der Mundhöhlenkörper 1402 in den beiden Bereichen 1575, 1575^{I} und 1580, 1580^{I} geschnitten dargestellt. Unmittelbar an die Anlagefläche 1404 schließt sich der erste Stielbogen 1490 an. In dem Bereich des ersten Stielbogens 1490 startet der transluzente Körper 1476, durch den die LEDs 1561, 1561^{I}, 1561^{V}, 1561^{VI}, 1561^{VII}, 1565, 1565^{I}, 1565^{V}, 1565^{VI}, 1565^{VII}, 1565^{VIII} ihr Licht 1415, 1415^{I} abgeben können. Somit gibt es ein endnahes Licht 1418 und ein mundschutznahes Licht 1417.

Der transluzente Körper 1476 ist durch die Kunststoffhülle 1482 gebildet, die Teil des Mundhöhlenkörpers 1402 ist. Im Inneren des Mundhöhlenkörpers 1402 gibt es den Lichtquellenträger, der als LED-Träger 1550 ausgestaltet ist. Der LED-Träger 1550 ist zur Aufnahme eines ersten LED-Strangs 1562 und eines zweiten LED-Strangs 1564 gestaltet. Innerhalb eines Strangs 1562, 1564 mit LEDs sind die LEDs 1561, 1561', 1561^{V}, 1561^{VI}, 1561^{VII}, 1565, 1565^{I}, 1565^{V}, 1565^{VI}, 1565^{VII}, 1565^{VIII} nicht alle gleichmäßig beabstandet. Zwischen LEDs, die im ersten Bereich 1575 des Mundhöhlenkörpers 1402 angesiedelt sind, gibt es einen ersten LED-Abstand 1569, der größer ist als ein zweiter LED-Abstand 1570, der im zweiten Bereich 1580, 1580^{I} vorzufinden ist. Der größere LED-Abstand 1569 beträgt 8 mm. Der kleine LED-Abstand 1570 beträgt weniger als die Hälfte, genauer gesagt 3 mm. Mehrere LEDs, wie z. B. die LEDs 1561^{V}, 1561^{VI}, 1561^{VII}, sind untereinander gleich beabstandet mit einem LED-Abstand 1570. Hiervon weicht der Abstand der LEDs 1561, 1561^{I} im Abschnitt unmittelbar vor der Anlagefläche 1404 ab. In dem sich oben befindlichen Bereich 1575, der auch als erster Bereich 1575 zu bezeichnen ist, und in dem sich unterhalb erstreckenden Bereich 1575^{I} sind die LEDs (z. B. LED 1561, 1561^{I}) alle gleich beabstandet. Auch die Abstände 1570 in dem zweiten Bereich 1580, 1580^{I} sind alle gleich.

Besonders vorteilhaft ist, wenn die LEDs 1561, 1561', 1561^{V}, 1561^{VI}, 1561^{VII} als LED-Band 1560 auf dem LED-Träger 1550 angeordnet sind. Der LED-Träger 1550 ist somit die Unterstützung für den ersten LED-Strang 1562 und die Halterung bzw. Unterstützung für den zweiten LED-Strang 1564. Der LED-Träger 1550 ist in seinem Endbereich mit einer Trägerrundung 1552 ausgestattet, sodass ein zusammenhängendes LED-Band 1560 auf dem LED-Träger 1550 beidseitig, genauer gesagt oben und unten, aufgelegt werden kann. Das LED-Band 1560 startet im ersten Stielbogen 1490 und läuft entlang des gesamten LED-Trägers 1550 über die Trägerrundung 1552 bis zur Zahnstützfläche 1484, die sich in Nachbarschaft zur Anlagefläche 1404 befindet.

Wird über den Einschaltaster 1501, der nur nach Ablauf einer Sperrzeit erneut betätigt werden kann, das Mundsteckstück 1401 eingeschaltet und leuchten die LEDs 1561, 1561^{I}, 1561^{V}, 1561^{VI}, 1561^{VII}, 1565, 1565^{I}, 1565^{V}, 1565^{VI}, 1565^{VII}, 1565^{VIII} im Bereich des Mundhöhlenkörpers 1402 durch die Kunststoffhülle 1482 hindurch, so stellt ein Betrachter fest, dass in dem zweiten Bereich 1580, 1580^{I} eine höhere Helligkeit abgegeben wird als in einem ersten Bereich 1575, 1575^{I}. Die LEDs 1561, 1561', 1561^{V}, 1561^{VI}, 1561^{VII}, 1565, 1565^{I}, 1565^{V}, 1565^{VI}, 1565^{VII}, 1565^{VIII} können aufgrund ihrer unterschiedlichen Ansteuerströme unterschiedlich hell leuchten. Außerdem sind in dem zweiten Abschnitt 1580, 1580^{I} mehr LEDs 1561^{V}, 1561^{VI}, 1561^{VII}, 1565^{V}, 1565^{VI}, 1565^{VII} angesiedelt. Beide Maßnahmen tragen dazu bei, dass im Bereich des Mundsteckstücks 1401, der für die Rachenbeleuchtung vorgesehen ist, ein stärkeres Licht leuchtet als im lippennahen Bereich. Die Lippen und die Zähne sollen im Bereich der Zahnstützfläche 1484 aufliegen. Im Bereich der Zahnstützfläche 1484 reicht eine geringere Beleuchtung im Vergleich zum rachennahen Bereich bzw. dem endnahen LED-Lichtbereich 1418 aus.

An zwei Seiten des LED-Trägers 1550 sind LED-Stränge 1562, 1564 vorhanden. Die Abstände 1569, 1570 zwischen den LEDs 1561, 1561^{I}, 1561^{V}, 1561^{VI}, 1561^{VII}, 1565, 1565^{I}, 1565^{V}, 1565^{VI}, 1565^{VII}, 1565^{VIII} sind zwischen dem ersten LED-Strang 1562 und dem zweiten LED-Strang 1564 gleich verteilt. Zwischen den LEDs 1561, 1561', 1565, 1565^{I}, die im Bereich der Bissfläche 1484 liegen, ist ein größerer Abstand 1569 als zwischen den LEDs 1561^{V}, 1561^{VI}, 1561^{VII}, 1565^{V}, 1565^{VI}, 1565^{VI}, die zur Ausleuchtung des Endnahenbereichs 1418 vorgesehen sind.

Durch die Versorgung mit verschiedenen Strömen, die an die LEDs 1561, 1561', 1561^{V}, 1561^{VI}, 1561^{VII}, 1565, 1565^{I}, 1565^{V}, 1565^{VI}, 1565^{VII}, 1565^{VIII} geliefert werden, können unterschiedliche Helligkeiten in unterschiedlichen Bereichen 1575, 1575^{I}, 1580, 1580^{I} erzeugt werden. Zu LEDs, wie die LEDs 1561^{V}, 1561^{VI}, 1561^{VII}, die heller leuchten sollen, kann ein Versorgungsstrom von 40 mA fließen. LEDs, die deutlich schwächer leuchten sollen, wie die LEDs 1561, 1561^{I} in Nachbarschaft zur Anlagefläche 1404, können mit Strom in der Größenordnung von 5 mA betrieben werden.

So ist es möglich, zielgerichtet Viren, Bakterien und Pilze abzutöten, insbesondere im Rachenbereich eines Nutzers, während mit deutlich niedrigeren Dosen in anderen Mundbereichen des Nutzers eine Bestrahlung durchgeführt wird.

Das Mundsteckstück 1601, das in der Figur 16 dargestellt ist, ähnelt in weiten Teilen dem Mundsteckstück 1401, das aus Figur 15 bekannt ist. Somit können die weiter oben stehenden Beschreibungen auf das Mundsteckstück 1601 gem. Figur 16 übertragen werden. Das Mundsteckstück 1601 gem. Figur 16 unterscheidet sich aber in Bezug auf die Platzierung der LEDs 1761, 1761^{I}, 1761^{II}, 1761^{III}, 1761^{IV}, 1761^{V}, 1761^{VI}, 1761^{VII}, 1765, 1765^{I}, 1765^{II}, 1765^{III}, 1765^{IV}, 1765^{V}, 1765^{VI} auf dem LED-Träger 1750.

Eine erste LED 1761, die nahe eines ersten Stielbogens 1690 angeordnet ist, weist einen ersten Abstand 1769 zu einer zweiten LED 1761^{I} auf. Der zweiten LED 1761^{I} folgen eine dritte LED 1761", eine vierte LED 1761^{III} eine fünfte LED 1761^{IV}, eine sechste LED 1761^{V} entlang eines zweiten Stielbogens 1691. Entlang eines dritten Stielbogens 1692 befinden sich eine siebte LED 1761^{VI} und eine achte LED 1761^{VII}. Zwischen der sechsten LED 1761^{V} und der siebten LED 1761^{VI} ist ein zweiter Abstand 1770 vorhanden. Der zweite Abstand 1770 beträgt ein Drittel einer Länge des ersten Abstands 1769. Von der ersten LED 1761 wird ein mundschutznahes Licht 1617 abgestrahlt. Von der achten LED 1761^{VII} wird ein endnahes Licht 1618 ausgesendet, das heller ist als das mundschutznahe LED-Licht 1617, weil die achte LED 1761^{VII} mit einem ca. 10% höheren Strom versorgt wird als die erste LED 1761. Auf dem transluzenten Körper 1676 des Mundhöhlenkörpers 1602 sind ein dunklerer Bereich 1775 und ein hellerer Bereich 1780 unterscheidbar. Somit wird gaumenseitig entlang der Stielbögen 1690, 1691, 1692 ein gaumenseitiges Licht 1615 abgestrahlt. Die LED-Platine 1760 ist um einen LED-Träger 1750 herum gelegt. Von zungenseitigen LEDs 1765, 1765^{I}, 1765", 1765^{III}, 1765^{IV}, 1765^{V}, 1765^{VI} wird zungenseitig Licht 1615^{I} abgestrahlt. Eine erste zungenseitige LED 1765 ist gegenüber der ersten gaumenseitigen LED 1761 und der zweiten gaumenseitigen LED 1761^{I} auf Lücke angeordnet, also deren Abstandbereich 1769 zugeordnet. Zwischen der ersten zungenseitigen LED 1765 und der zweiten zungenseitigen LED 1765^{I} ist ein Abstand 1769^{I} vorhanden. Zwischen der dritten zungenseitigen LED 1765^{II} und der vierten zungenseitigen LED 1765^{III} ist ein Abstand 1774 vorhanden. Zwischen der sechsten zungenseitigen LED 1765^{V} und der siebten zungenseitigen LED 1765^{VI} ist ein Abstand 1770^{I} vorhanden. Der erste Abstand 1769^{I} und der dritte Abstand 1770^{I} sind etwa gleich groß. Der zweite Abstand 1774 ist um ein Drittel größer als der erste Abstand 1769^{I}, weil ein Stielmittenbogen 1696 des Mundhöhlenkörpers 1602 eine konkave Krümmung aufweist, durch die sich Abstrahlungsbereiche der dritten LED 1765^{I} und der vierten LED 1765^{II} überschneiden. Wie bei dem gaumenseitigen Stielansatzbogen 1690 ist auch bei dem zungenseitigen Stielansatzbogen 1695 ein schwächeres Licht 1617^{I} aus der ersten, zungenseitigen LED 1765 zur Behandlung ausreichend. Von der siebten LED 1765^{VI} wird ein im Vergleich zu dem Licht 1617^{I} stärkeres bzw. helleres Licht 1618^{I} durch erhöhte Stromzufuhr zu der siebten LED 1765^{VI} abgestrahlt, sodass von einem mundhöhlenseitigen Ende 1699 des Mundhöhlenkörpers 1602 ein Eingangsbereich zum Rachen ausreichend mit Licht bestrahlt wird. Anders gesagt, liegt an dem zungenseitigen Stielendbogen 1697 ein zweiter Helligkeitsbereich 1780^{I} vor, zu dem im Vergleich ein erster Helligkeitsbereich 1775^{I}, der dem zungenseitigen Stielansatzbogen 1695 zugeordnet ist, dunkler erscheint. Das von einer LED 1761, 1761^{I}, 1761^{II}, 1761^{III} 1761^{IV}, 1761^{V}, 1761^{VI}, 1761^{VII} sowie 1765, 1765^{I}, 1765^{II}, 1765^{III}, 1765^{IV}, 1765^{V} 1765^{VI} jeweils abgegebene Licht, wie das Licht 1617, 1617', 1618, 1618^{I}, ist jeweils durch Stromzufuhr über eine Steuerplatine, die sich im Inneren eines kastenförmigen Körpers 1660 befindet, festgelegt. Der kastenförmige Körper 1660 weist ein Gehäuse 1662 auf, das an einen Mundschutz 1668 angeschlossen ist. Von dem Mundschutz 1668 bis zum siebten Stielbogen 1699 erstreckt sich der Mundhöhlenkörper 1602, der den LED-Träger 1750 enthält. Ein Einschalttaster 1701 an dem Gehäuse 1662 dient dazu, die fünfzehn LEDs 1761, 1761^{I}, 1761^{II}, 1761^{III}, 1761^{IV}, 1761^{V}, 1761^{VI}, 1761^{VII} sowie 1765, 1765^{I}, 1765", 1765^{III}, 1765^{IV}, 1765^{V} 1765^{VI} des Mundsteckstücks 1601, also acht gaumenseitige und sieben zungenseitige, gemeinsam ein- bzw. auszuschalten.

### Bezugszeichenliste

- 1, 201, 401, 601, 801,: Mundsteckstück
- 1001, 1201, 1401, 1601 2, 202, 402, 602, 1402,: Mundhöhlenkörper
- 1602 3, 203: Längserstreckung, insbesondere des Mundsteckstücks
- 4, 204, 404, 1404: Anlagefläche
- 5, 205, 605: Auflage, insbesondere Lichtreflexionsfläche
- 6, 406: Lichtquelle
- 7, 407: Energiequelle, insbesondere Akkumulator
- 8: LED
- 9, 409: Zeitsteuerung
- 10, 210: Lichtabgabebereich
- 11, 611, 1411: knopfartiger Körper
- 12, 212: elektrischer Anschluss
- 13, 213: erste Halbschale
- 14, 214: zweite Halbschale
- 15, 1415, 1415^{I}, 1615,: Licht
- 1615^{I} 16: Kabel
- 1417, 1617, 1617^{I}: mundschutznahes LED-Licht
- 1418, 1618, 1618^{I}: endnahes LED-Licht, insbesondere Licht zur Behandlung eines Rachenbereichs
- 20, 20^{I}: Mundhöhle, insbesondere Mundinnenraum
- 21, 21^{I}: obere Schneidezähne
- 22, 22': untere Schneidezähne
- 23, 23^{I}: Oberlippe
- 23^{II}: Amorbogen
- 24, 24^{I}: Unterlippe
- 25, 25^{I}: Zunge
- 26, 26^{I}: Gaumen, insbesondere harter Gaumen mit Wölbung
- 28: Gaumen, insbesondere weicher Gaumen mit Wölbung
- 30: Gaumenzäpfchen
- 32: Zungenrücken, insbesondere anterodorsaler Bereich einer Zungenwölbung
- 34: Zungenrücken, insbesondere posterodorsaler Bereich einer Zungenwölbung
- 36: sublaminaler Bereich
- 38: Oberkiefer
- 40: Unterkiefer
- 42: Rachen
- 44: Nasenhöhle
- 46: Nasenrachen
- 48: Kehldeckel
- 50: Luftröhre
- 458, 1258, 1458: blockartiger Körper
- 260, 860, 1660: kastenartiger Körper
- 262, 462, 862, 1062,: Gehäuse
- 1462, 1662 263: Anschlusskante
- 264,464: Griff
- 465: Reflektorfläche
- 266: Längserstreckung des kastenartigen Körpers
- 268, 468, 668, 1468,: Mundschutz
- 1668 269: Lippenbezugspunkt
- 270: Breite des Mundschutzes
- 271: Anschlusslippe
- 272: Höhe des Mundschutzes
- 273, 473: Dehnungsmulde
- 274: Breite des kastenartigen Körpers
- 275: Durchlüftungsöffnung, insbesondere mit Keimfilter ausgestattete Öffnung
- 276, 476, 676, 1476,: transluzenter Körper
- 1676 278, 478: bogenförmiger Stiel
- 280, 480: Lippenanschlusskörper
- 282, 482, 1482: Kunststoffhülle, insbesondere Hülle des Mundhöhlenkörpers
- 284, 284', 1484: Zahnstützfläche, insbesondere Bissfläche
- 286: Verlängerungsbereich
- 290, 490, 1490, 1690: erster Stielbogen, insbesondere gaumenseitiger Stielansatzbogen
- 291, 491, 1691: zweiter Stielbogen, insbesondere gaumenseitiger Stielmittenbogen
- 292, 492, 1692: dritter Stielbogen, insbesondere gaumenseitiger Stielendbogen
- 295, 1695: vierter Stielbogen, insbesondere zungenseitiger Stielansatzbogen
- 296, 1696: fünfter Stielbogen, insbesondere zungenseitiger Stielmittenbogen
- 297, 1697: sechster Stielbogen, insbesondere zungenseitiger Stielendbogen
- 299, 1699: siebter Stielbogen, insbesondere mundhöhlenseitiges Ende des Mundhöhlenkörpers
- 301, 501, 701, 1501,: Einschalttaster
- 1701 502: Hebelführung
- 303: Tasterfeder
- 504: Schalter, insbesondere Schalter mit Zeitsteuerung, wie ein Timer
- 305, 505: USB-Buchse
- 510: Batteriefach bzw. Akkumulatorfach
- 512: Tasteranschlag
- 520, 920, 1320: Steuerplatine
- 530, 930, 1130, 1330: Abschlussplatte
- 532: Dichtfläche
- 334, 534, 534^{I}, 534^{II}: Kippnase bzw. Klippnase
- 536: Dichtkante
- 540: Inneres, insbesondere des Mundhöhlenkörpers
- 550, 950, 1150, 1350,: LED-Träger
- 1550, 1750 1151, 1151^{I}, 1351,: Seitenschutz
- 1351^{I} 1152, 1352, 1552: Trägerrundung
- 1153: Steckkontakt
- 1154: Kontaktführung
- 1355: Einstecköffnung
- 556: Anschraubverbinder
- 957,1157,1357: Clipverbinder
- 558, 958, 1158: Steckverbinder
- 959, 959^{I}, 1159, 1159^{I}: Clip, insbesondere seitlicher Clip
- 560, 1160, 1360, 1560,: LED-Band, insbesondere LED-Platine
- 1760 1360^{I}: Platinenmittenbereich, insbesondere elastischer Rundungsbereich
- 961, 961^{I}, 961^{II}, 961^{III},: LED
- 961^{IV}, 961^{V}, 961^{VI}, 961^{VII}, 961^{VIII}, 1361,:
- 1361^{I}, 1361^{II}, 1361^{III} 1361^{IV}, 1361^{V}, 1361^{VI},:
- 1561, 1561', 1561^{V}, 1561^{VI}, 1561^{VII}, 1761,:
- 1761^{I}, 1761^{II}, 1761^{III} 1761^{IV}, 1761^{V}, 1761^{VI}, 1761^{VII}:
- 562, 1562: erster LED-Strang, insbesondere obere LEDs
- 1163, 1363: Platinenendbereich
- 564, 1564: zweiter LED-Strang, insbesondere untere LEDs
- 1565, 1565^{I},: LED
- 1565^{V}, 1565^{VI}, 1565^{VII},:
- 1565^{VIII}, 1765, 1765^{I},:
- 1765^{II}, 1765^{III}, 1765^{IV},:
- 1765^{V}, 1765^{VI}:
- 566, 1366: Kontaktfeld mit mehreren Kontakten, insbesondere LED-Stromversorgungskontakte
- 1367, 1367^{I}: LED-Paar
- 1368: Rundungs-LED-Paar
- 1369, 1569, 1769,: erster LED-Abstand
- 1769^{I} 1370, 1570, 1770,: zweiter LED-Abstand
- 1770^{I} 571, 573: Blaulicht-LED
- 572: Blaulicht-LED, insbesondere LED zur Bestrahlung des sublaminalen Bereichs
- 1774: LED-Abstand, insbesondere Abstand von zwei zungenseitigen LEDs im Mittenbereich des Mundhöhlenkörpers
- 1575, 1575^{I}, 1775,: erster Bereich, insbesondere Bereich mit einer ersten Helligkeit,
- 1775^{I}: wie ein dunklerer Lichtabgabebereich des Mundhöhlenkörpers
- 577, 578: UV-C-Licht-LED
- 579: Laser-LED
- 1580, 1580^{I}, 1780,: zweiter Bereich, insbesondere Bereich mit einer zweiten Helligkeit,
- 1780^{I}: wie ein hellerer Lichtabgabebereich des Mundhöhlenkörpers
- 581: Temperaturüberwachung, insbesondere Temperatursensor
- 583: Mikrolinsenoptik
- 589, 589^{I}: Betriebszustands-LED, vorzugsweise Seiten-LED
- 591, 591^{I}: Lichtkanal
- 593: Betriebszustandsanzeige, insbesondere in Form eines transluzenten Fensters

## Patentansprüche

1. Mundsteckstück (201, 401, 601, 801, 1001, 1201, 1401, 1601)
mit einem Mundhöhlenkörper (202, 402, 602, 1402, 1602) zur
Aufnahme des Mundhöhlenkörpers (202, 402, 602, 1402, 1602) im Mundraum (20, 20^{I}) eines Menschen oder Tiers,
der ein wenigstens abschnittsweise, vorzugsweise vollständig, transluzenter Körper (276, 476, 676, 1476, 1676) ist,
durch den eine Abgabe eines Lichts (1415, 1415^{I}, 1417, 1418, 1615, 1615^{I}, 1617, 1617^{I}, 1618, 1618^{I})
mit einer bakterizid und/oder viruzid und/oder fungizid wirkenden Wellenlänge ermöglicht wird,
**dadurch gekennzeichnet, dass**
der Mundhöhlenkörper (202, 402, 602, 1402, 1602) zur dentogingivalen und lingualen Aufnahme stabartig oder zapfenartig, in einem mittleren Bereich im Vergleich zu seinen Endbereichen angehoben und leicht geschwungen ist, um einer Gaumenwölbung und einer Zungenwölbung nachgeführt zu sein,
dass das Mundsteckstück (201, 401, 601, 801, 1001, 1201, 1401, 1601) mit einer quer zu einer Längserstreckung (203) des Mundsteckstücks (201, 401, 601, 801, 1001, 1201, 1401, 1601) ausgerichteten Anlagefläche (204, 404,1404) ausgestattet ist,
die mit einer Wölbung eines Kreisbogenausschnitts oder zylindermantelartig gewölbt, konkav dem Mundhöhlenkörper (202, 402, 602, 1402, 1602) zugewandt zur vollflächigen Auflage (205, 605) zumindest auf einem Endbereich eines Amorbogens (23^{II}) eines menschlichen Gesichts gestaltet ist,
und dass in einem Inneren (540) des Mundhöhlenkörpers (202, 402, 602, 1402, 1602) wenigstens eine in einem Blaulichtbereich Licht abgebende Lichtquelle (406) zu einer wenigstens posterioren Mundhöhlenbeleuchtung sitzt.

2. Mundsteckstück (201, 401, 601, 801, 1001, 1201, 1401, 1601) nach Anspruch 1,
wobei das Mundsteckstück (201, 401, 601, 801, 1001, 1201, 1401, 1601) mit einer Energiequelle, z. B. über eine USB-Buchse (305, 505), verbindbar ist oder eine Energiequelle (407) umfasst, insbesondere für eine Niedervoltversorgung der Lichtquelle (406).

3. Mundsteckstück (201, 401, 601, 801, 1001, 1201, 1401, 1601) nach einem der vorhergehenden Ansprüche,
wobei die Lichtquelle (406) eine Blaulicht-Lichtquelle ist,
die sich aus mehreren LEDs (560, 562, 564, 961, 961^{I}, 961", 961^{III}, 961^{IV}, 961^{V}, 961^{VI}, 961^{VII}, 961^{VIII}, 1361, 1367, 1368, 1561, 1565, 1761, 1765), insbesondere RGB-LEDs und/oder Blaulicht-LEDs (571, 572, 573) bildet,
insbesondere aus einem Satz (560, 562, 564) unterschiedlicher LEDs wie einer UV-Licht-LED (577, 578) und mehreren Blaulicht-LEDs (571, 572, 573) und/oder aus mehreren typgleichen LEDs (961, 961^{I}, 961", 961^{III}, 961^{IV}, 961^{V}, 961^{VI}, 961^{VII}, 961^{VIII}, 1361, 1367, 1368, 1561, 1761),
durch die vorzugsweise unterschiedliche Lichtdichten durch Licht (1415, 1415^{I}, 1417, 1418, 1615, 1615^{I}, 1617, 1617^{I}, 1618, 1618^{I}) aus verschiedenen Bereichen (210, 1575, 1575^{I}, 1580, 1580^{I} 1775, 1775^{I}, 1780, 1780^{I}) des Mundhöhlenkörpers (202, 402, 602, 1402, 1602) herstellbar sind, z. B. durch von einer LED zu einer anderen LED (1561, 1561^{VII}, 1565, 1565^{VIII}; 1761, 1761^{VII}; 1765, 1765^{VI}) abweichende Versorgungsströme und/oder z. B. durch in einem ersten Bereich dichter angeordnete (1570, 1770, 1770^{I}) und in einem zweiten Bereich weiter beabstandete (1569, 1769, 1769^{I}, 1774) LEDs.

4. Mundsteckstück (201, 401, 601, 801, 1001, 1201, 1401, 1601) nach einem der vorhergehenden Ansprüche,
wobei das Mundsteckstück (201, 401, 601, 801, 1001, 1201, 1401, 1601) eine Zeitsteuerung (409) und/oder eine Temperaturüberwachung (581) umfasst, durch die eine Beleuchtungsdauer, während der die Lichtquelle (406) Blaulicht abgibt, zeitlich und/oder leistungsmäßig begrenzt wird,
insbesondere mit zwei unterschiedlichen LED-Betriebsphasen,
vorzugsweise einer längeren für LEDs (571, 572, 573),
die im sichtbaren Wellenspektrum Licht ausstrahlen, und
einer hierzu kürzeren für wenigstens eine LED (577, 578),
die eine Wellenfront oder Strahlung jenseits des sichtbaren Wellenspektrums aussendet.

5. Mundsteckstück (201, 401, 601, 801, 1001, 1201, 1401, 1601) nach einem der vorhergehenden Ansprüche,
wobei das Mundsteckstück (201, 401, 601, 801, 1001, 1201, 1401, 1601) ein Batterie- bzw. Akkumulatorfach (510) im außerhalb eines menschlichen Körpers anzusiedelnden Bereich des Mundsteckstücks (201, 401, 601, 801, 1001, 1201, 1401, 1601) umfasst, in das ein Akkumulator oder eine Batterie zur elektrischen Versorgung der Lichtquelle (406) einlegbar oder eingebaut, z. B. eingelötet, ist.

6. Mundsteckstück (201, 401, 601, 801, 1001, 1201, 1401, 1601) nach einem der vorhergehenden Ansprüche,
wobei die Lichtquelle (406), insbesondere neben wenigstens einer weiteren Wellenlänge, für eine Abgabe von Licht (1415, 1415^{I}, 1417, 1418, 1615, 1615^{I}, 1617, 1617^{I}, 1618, 1618^{I}) in einem Wellenlängenbereich zwischen 400 nm und 490 nm, vorzugsweise von Licht mit einer Wellenlänge von ca. 405 nm und/oder einem Licht mit einer Wellenlänge von ca. 453 nm und/oder einem Licht mit einer Wellenlänge von ca. 470 nm, gestaltet ist.

7. Mundsteckstück (201, 401, 601, 801, 1001, 1201, 1401, 1601) nach einem der Ansprüche 1 bis 7,
wobei die Lichtquelle (406) für eine Abgabe von Licht bzw. Strahlung in einem UV-Wellenlängenbereich, z. B. von 222 nm, zusätzlich zum Blaulichtbereich, gestaltet ist.

8. Mundsteckstück (201, 401, 601, 801, 1001, 1201, 1401, 1601) nach einem der vorhergehenden Ansprüche,
wobei der Mundhöhlenkörper (202, 402, 602, 1402, 1602) für ein gerichtet von der Lichtquelle (406) abgestrahltes Licht ausgestaltet ist, wobei vorzugsweise ein Spitzenbereich des Mundhöhlenkörpers (202, 402, 602, 1402, 1602) als Lichtabgabebereich (210, 1580, 1580^{I}, 1780, 1780^{I}) vorgesehen ist.

9. Mundsteckstück (201, 401, 601, 801, 1001, 1201, 1401, 1601) nach einem der vorhergehenden Ansprüche,
wobei das Mundsteckstück (201, 401, 601, 801, 1001, 1201, 1401, 1601) einen knopfartigen Körper (611), einen kastenartigen Körper (260, 860, 1660) und/oder einen blockartigen Körper (458, 1258, 1458), insbesondere als Griff (264, 464), hat,
der jenseits der Anlagefläche (204, 404) angesiedelt ist,
wobei insbesondere der Körper, wie knopfartiger, kastenartiger oder blockartiger Körper (260, 458, 611), mit einem elektrischen Anschluss (212), wie z. B. einem Kabel, eine Buchse (305, 505) oder einem Stecker, ausgestattet ist.

10. Mundsteckstück (201, 401, 601, 801, 1001, 1201, 1401, 1601) nach einem der vorhergehenden Ansprüche,
wobei der Mundhöhlenkörper (202, 402, 602, 1402, 1602) nachgiebig und/oder flüssigkeitsdicht ist, z. B. aufgrund einer Kunststoffhülle (282, 482) wie z. B. aus einem Silikon oder z. B. aufgrund einer Latex-Hülle.

11. Mundsteckstück (201, 401, 601, 801, 1001, 1201, 1401, 1601) nach einem der vorhergehenden Ansprüche,
wobei das Mundsteckstück (201, 401, 601, 801, 1001, 1201, 1401, 1601) einen aus zwei Halbschalen (213, 214), z. B. oval gestalteten Querkörper, symmetrisch gestalteten Mundhöhlenkörper (202, 402, 602, 1402, 1602) hat,
der insbesondere aufgrund von Druck oder Unterdruck eine formadaptive Oberfläche zu einer Mundhöhle (20, 20^{I}) eines Nutzers ausbilden kann.

12. Mundsteckstück (201, 401, 601, 801, 1001, 1201, 1401, 1601) nach einem der vorhergehenden Ansprüche,
wobei der Mundhöhlenkörper (202, 402, 602, 1402, 1602) verschiebbar ist, insbesondere verlängerbar und/oder deplatzierbar ist in Bezug auf einen Lippenbezugspunkt (269) des Mundsteckstücks (201, 401, 601, 801, 1001, 1201, 1401, 1601),
oder der Mundhöhlenkörper (202, 402, 602, 1402, 1602),
insbesondere durch ein Aufspreizen oder ein Zusammendrücken von haltenden Klippnasen (534", 959, 959^{I}, 1159, 1159^{I}), z. B. durch zwei entgegengesetzt gerichtete Bewegungen, auswechselbar ist,
wodurch vorzugsweise das Mundsteckstück (201, 401, 601, 801, 1001, 1201, 1401, 1601) in seiner Länge (203) veränderbar ist.

13. Mundsteckstück (201, 401, 601, 801, 1001, 1201, 1401, 1601) nach einem der vorhergehenden Ansprüche,
wobei an dem Mundsteckstück (201, 401, 601, 801, 1001, 1201, 1401, 1601), insbesondere in einem seitlichen Bereich des Körpers, ein Einschaltknopf, z. B. ein zeitlich verriegelnder Einschalttaster (301, 501, 701, 1501, 1701), vorhanden ist.

14. Mundsteckstück (201, 401, 601, 801, 1001, 1201, 1401, 1601) nach einem der vorhergehenden Ansprüche,
wobei das Licht (1415, 1415^{I}, 1417, 1418, 1615, 1615^{I}, 1617, 1617^{I}, 1618, 1618^{I}) durch mehrere LEDs (571, 572, 573, 577, 578, 579, 961, 1361, 1367, 1368, 1561, 1565, 1761, 1765) erzeugt eine Wellenlänge im Violettlichtbereich und/oder im Ultraviolettlichtbereich, zusätzlich zum Blaulichtbereich, besitzt.

## Claims

1. Mouth insertion piece (201, 401, 601, 801, 1001, 1201, 1401, 1601) with an oral cavity body (202, 402, 602, 1402, 1602)
for accommodating the oral cavity body (202, 402, 602, 1402, 1602) in an oral cavity (20, 20^{I}) of a human or animal,
the oral cavity body being an at least partially, preferably entirely, translucent body (276, 476, 676, 1476, 1676),
through which light (1415, 1415^{I}, 1417, 1418, 1615, 1615^{I}, 1617, 1617^{I}, 1618, 1618^{I}) with a wavelength that has a bactericidal and/or virucidal and/or fungicidal effect can be emitted,
**characterized in that**
the oral cavity body (202, 402, 602, 1402, 1602) is rod-shaped or cone-shaped in order to be accommodated in a dentogingival and lingual area, is elevated in a middle region compared to its end regions, and is slightly curved in order to follow a palate curvature and a tongue curvature,
**in that** the mouth insertion piece (201, 401, 601, 801, 1001, 1201, 1401, 1601) is equipped with an abutment surface (204, 404, 1404) oriented transversely to a longitudinal extension (203) of the mouth insertion piece (201, 401, 601, 801, 1001, 1201, 1401, 1601),
said abutment surface being curved with a curvature of a circular arc segment or in the manner of a cylinder and being concave in a direction facing toward the oral cavity body (202, 402, 602, 1402, 1602) for full-surface bearing (205, 605) at least on an end region of a Cupid's bow (23") of a human face,
and **in that** at least one light source (406) that emits light in a blue light range in order to provide illumination of at least the posterior region of the oral cavity is seated in an interior (540) of the oral cavity body (202, 402, 602, 1402, 1602).

2. Mouth insertion piece (201, 401, 601, 801, 1001, 1201, 1401, 1601) according to claim 1,
wherein
the mouth insertion piece (201, 401, 601, 801, 1001, 1201, 1401, 1601) can be connected to a power source, for example via a USB socket (305, 505), or includes a power source (407), in particular for supplying a low voltage to the light source (406).

3. Mouth insertion piece (201, 401, 601, 801, 1001, 1201, 1401, 1601) according to any one of the preceding claims,
wherein
the light source (406) is a blue light source,
which is formed of a plurality of LEDs (560, 562, 564, 961, 961^{I}, 961", 961^{III}, 961^{IV}, 961^{V}, 961^{VI}, 961^{VII}, 961^{VIII}, 1361, 1367, 1368, 1561, 1565, 1761, 1765), in particular RGB LEDs and/or blue-light LEDs (571, 572, 573),
in particular of a set (560, 562, 564) of different LEDs, such as one UV-light LED (577, 578) and a plurality of blue-light LEDs (571, 572, 573),
and/or of a plurality of identical LEDs (961, 961^{I}, 961", 961^{III}, 961^{IV}, 961^{V}, 961^{VI}, 961^{VII}, 961^{VIII}, 1361, 1367, 1368, 1561, 1761),
by which preferably different light densities can be produced by light (1415, 1415^{I}, 1417, 1418, 1615, 1615^{I}, 1617, 1617^{I}, 1618, 1618^{I}) from different regions (210, 1575, 1575^{I}, 1580, 1580^{I}, 1775, 1775^{I}, 1780, 1780^{I}) of the oral cavity body (202, 402, 602, 1402, 1602), for example by supply currents differing from one LED to another LED (1561, 1561^{VII}, 1565, 1565^{VIII}; 1761, 1761^{VII}; 1765, 1765^{VI}) and/or for example by LEDs (1570, 1770, 1770^{I}) arranged more densely in a first region and LEDs (1569, 1769, 1769^{I}, 1774) spaced further apart in a second region.

4. Mouth insertion piece (201, 401, 601, 801, 1001, 1201, 1401, 1601) according to any one of the preceding claims,
wherein
the mouth insertion piece (201, 401, 601, 801, 1001, 1201, 1401, 1601) includes a time control (409) and/or a temperature monitoring (581), by which an illumination duration, during which the light source (406) emits blue light, is limited in terms of time and/or power,
in particular by having two different LED operating phases,
preferably a longer operating phase for LEDs (571, 572, 573)
that emit light in the visible wave spectrum, and
a shorter operating phase for at least one LED (577, 578)
that emits a wavefront or radiation beyond the visible wave spectrum.

5. Mouth insertion piece (201, 401, 601, 801, 1001, 1201, 1401, 1601) according to any one of the preceding claims,
wherein
the mouth insertion piece (201, 401, 601, 801, 1001, 1201, 1401, 1601) includes a battery or accumulator compartment (510) in a region of the mouth insertion piece (201, 401, 601, 801, 1001, 1201, 1401, 1601) that is to be located outside a human body, into which an accumulator or a battery for supplying electrical power to the light source (406) can be inserted or installed, for example soldered.

6. Mouth insertion piece (201, 401, 601, 801, 1001, 1201, 1401, 1601) according to any one of the preceding claims,
wherein
the light source (406), in particular in addition to at least one further wavelength, is designed to emit light (1415, 1415^{I}, 1417, 1418, 1615, 1615^{I}, 1617, 1617^{I}, 1618, 1618^{I}) in a wavelength range between 400 nm and 490 nm, preferably light with a wavelength of around 405 nm and/or light with a wavelength of around 453 nm and/or light with a wavelength of around 470 nm.

7. Mouth insertion piece (201, 401, 601, 801, 1001, 1201, 1401, 1601) according to any one of claims 1 to 7,
wherein
the light source (406) is designed to emit light or radiation in a UV wavelength range, for example of 222 nm, in addition to the blue light range.

8. Mouth insertion piece (201, 401, 601, 801, 1001, 1201, 1401, 1601) according to any one of the preceding claims,
wherein
the oral cavity body (202, 402, 602, 1402, 1602) is designed for light emitted directionally by the light source (406), wherein preferably a tip region of the oral cavity body (202, 402, 602, 1402, 1602) is provided as a light-emitting region (210, 1580, 1580^{I}, 1780, 1780^{I}).

9. Mouth insertion piece (201, 401, 601, 801, 1001, 1201, 1401, 1601) according to any one of the preceding claims,
wherein
the mouth insertion piece (201, 401, 601, 801, 1001, 1201, 1401, 1601) has a knob-shaped body (611), a box-shaped body (260, 860, 1660) and/or a block-shaped body (458, 1258, 1458), in particular in the form of a handle (264, 464),
which is located on the other side of the abutment surface (204, 404),
wherein in particular the body, such as the knob-shaped, box-shaped or block-shaped body (260, 458, 611), is equipped with an electrical connector (212), such as for example a cable, a socket (305, 505) or a plug.

10. Mouth insertion piece (201, 401, 601, 801, 1001, 1201, 1401, 1601) according to any one of the preceding claims,
wherein
the oral cavity body (202, 402, 602, 1402, 1602) is resilient and/or liquid-tight, for example on account of a plastic cover (282, 482), such as for example made of a silicone, or for example on account of a latex cover.

11. Mouth insertion piece (201, 401, 601, 801, 1001, 1201, 1401, 1601) according to any one of the preceding claims,
wherein
the mouth insertion piece (201, 401, 601, 801, 1001, 1201, 1401, 1601) has an oral cavity body (202, 402, 602, 1402, 1602) formed symmetrically from two half-shells (213, 214), for example oval shaped transverse bodies,
which oral cavity body can in particular form a shape-adaptive surface to the oral cavity (20, 20^{I}) of a user under the effect of pressure or a vacuum.

12. Mouth insertion piece (201, 401, 601, 801, 1001, 1201, 1401, 1601) according to any one of the preceding claims,
wherein
the oral cavity body (202, 402, 602, 1402, 1602) is movable,
in particular is extendable and/or displaceable in relation to a lip reference point (269) of the mouth insertion piece (201, 401, 601, 801, 1001, 1201, 1401, 1601),
or the oral cavity body (202, 402, 602, 1402, 1602) is interchangeable,
in particular by spreading apart or pressing together retaining clip lugs (534", 959, 959^{I}, 1159, 1159^{I}), for example by two movements directed in opposite directions,
as a result of which preferably the length (203) of the mouth insertion piece (201, 401, 601, 801, 1001, 1201, 1401, 1601) can be varied.

13. Mouth insertion piece (201, 401, 601, 801, 1001, 1201, 1401, 1601) according to any one of the preceding claims,
wherein
a power switch, for example a time-locking switch-on button (301, 501, 701, 1501, 1701), is provided on the mouth insertion piece (201, 401, 601, 801, 1001, 1201, 1401, 1601), in particular in a side region of the body.

14. Mouth insertion piece (201, 401, 601, 801, 1001, 1201, 1401, 1601) according to any one of the preceding claims,
wherein
the light (1415, 1415^{I}, 1417, 1418, 1615, 1615^{I}, 1617, 1617^{I}, 1618, 1618^{I}) generated by a plurality of LEDs (571, 572, 573, 577, 578, 579, 961, 1361, 1367, 1368, 1561, 1565, 1761, 1765) has a wavelength in the violet light range and/or in the ultraviolet light range, in addition to the blue light range.

## Revendications

1. Embout à insérer dans la bouche (201, 401, 601, 801, 1001, 1201, 1401, 1601) avec un corps de cavité buccale (202, 402, 602, 1402, 1602) pour recevoir le corps de cavité buccale (202, 402, 602, 1402, 1602) dans une cavité buccale (20, 20') d'un être humain ou d'un animal, corps qui est un corps au moins partiellement, de préférence entièrement translucide (276, 476, 676, 1476, 1676), par lequel il est rendu possible de délivrer une lumière (1415, 1415', 1417, 1418, 1615, 1615^{I}, 1617, 1617^{I}, 1618, 1618^{I}) avec une longueur d'onde qui a un effet bactéricide et/ou virucide et/ou fongicide,
**caractérisé en ce que**
le corps de cavité buccale (202, 402, 602, 1402, 1602) est relevé en forme de tige ou de tenon dans une zone centrale par comparaison avec ses zones d'extrémité et est légèrement galbé pour recevoir une région dento-gingivale et linguale pour suivre une courbure du palais et une courbure de la langue,
que l'embout à insérer dans la bouche (201, 401, 601, 801, 1001, 1201, 1401, 1601) est équipé d'une surface d'appui (204, 404, 1404), orientée transversalement par rapport à une extension longitudinale (203) de l'embout à insérer dans la bouche (201, 401, 601, 801, 1001, 1201, 1401, 1601), qui est configurée avec une courbure d'une découpe en arc de cercle ou voûtée en forme de type d'enveloppe cylindrique, tournée concave vers le corps de cavité buccale (202, 402, 602, 1402, 1602) pour un appui sur toute la surface (205, 605) au moins sur une zone d'extrémité de l'arc de Cupidon (23") d'un visage humain
et qu'au moins une source lumineuse (406) qui émet de la lumière dans le spectre de lumière bleue se trouve dans un intérieur (540) du corps de cavité buccale (202, 402, 602, 1402, 1602) pour un éclairage au moins postérieur de la cavité buccale.

2. Embout à insérer dans la bouche (201, 401, 601, 801, 1001, 1201, 1401, 1601) selon la revendication 1, l'embout à insérer dans la bouche (201, 401, 601, 801, 1001, 1201, 1401, 1601) pouvant être relié à une source d'énergie, par exemple par un connecteur USB (305, 505), ou comprenant une source d'énergie (407), en particulier pour une alimentation basse tension de la source lumineuse (406).

3. Embout à insérer dans la bouche (201, 401, 601, 801, 1001, 1201, 1401, 1601) selon l'une des revendications précédentes, la source lumineuse (406) étant une source lumineuse de lumière bleue qui est formée par plusieurs LED (560, 562, 564, 961, 961^{I}, 961^{II}, 961^{III}, 961^{IV}, 961^{V}, 961^{VI}, 961^{VII}, 961^{VIII}, 1361, 1367, 1368, 1561, 1565, 1761, 1765), en particulier des LED RGB et/ou des LED de lumière bleue (571, 572, 573), en particulier par un jeu (560, 562, 564) de différentes LED comme une LED de lumière UV (577, 578) et plusieurs LED de lumière bleue (571, 572, 573) et/ou par plusieurs LED de même type (961, 961^{I}, 961^{II}, 961^{III}, 961^{IV}, 961^{V}, 961^{VI}, 961^{VII}, 961^{VIII}, 1361, 1367, 1368, 1561, 1761), par lesquelles de préférence des densités de lumière différentes peuvent être réalisées par de la lumière (1415, 1415^{I}, 1417, 1418, 1615, 1615^{I}, 1617, 1617^{l}, 1618, 1618^{I}) de différentes régions (210, 1575, 1575^{I}, 1580, 1580^{I} 1775, 1775^{I}, 1780, 1780^{I}) du corps de cavité buccale (202, 402, 602, 1402, 1602), par exemple par des courants d'alimentation qui diffèrent d'une LED à une autre LED (1561, 1561^{VII}, 1565, 1565^{VIII}; 1761, 1761^{VII}, 1765, 1765^{VI}) et/ou par exemple par des LED placées plus proches dans une première région (1570, 1770, 1770') et davantage espacées dans une seconde région (1569, 1769, 1769^{I}, 1774).

4. Embout à insérer dans la bouche (201, 401, 601, 801, 1001, 1201, 1401, 1601) selon l'une des revendications précédentes, l'embout à insérer dans la bouche (201, 401, 601, 801, 1001, 1201, 1401, 1601) comprenant une commande du temps (409) et/ou une surveillance de la température (581) par laquelle une durée d'éclairage pendant laquelle la source lumineuse (406) émet de la lumière bleue est limitée dans le temps et/ou en matière de performance, en particulier avec deux phases différentes de fonctionnement de LED, de préférence une phase plus longue pour des LED (571, 572, 573) qui émettent de la lumière dans le spectre d'ondes visibles et une phase plus courte par rapport à celle-ci pour au moins une LED (577, 578) qui émet un front d'ondes ou un rayonnement au-delà du spectre d'ondes visibles.

5. Embout à insérer dans la bouche (201, 401, 601, 801, 1001, 1201, 1401, 1601) selon l'une des revendications précédentes, l'embout à insérer dans la bouche (201, 401, 601, 801, 1001, 1201, 1401, 1601) comprenant un compartiment à pile ou à accumulateur (510) dans une zone de l'embout à insérer dans la bouche (201, 401, 601, 801, 1001, 1201, 1401, 1601) qui doit être placée à l'extérieur d'un corps humain, compartiment dans lequel un accumulateur ou une pile doit être insérée ou montée, par exemple en étant brasée, pour l'alimentation électrique de la source lumineuse (406).

6. Embout à insérer dans la bouche (201, 401, 601, 801, 1001, 1201, 1401, 1601) selon l'une des revendications précédentes, la source lumineuse (406) étant agencée, en particulier en plus d'au moins une autre longueur d'onde, pour une émission de lumière (1415, 1415^{I}, 1417, 1418, 1615, 1615^{I}, 1617, 1617^{I}, 1618, 1618^{I}) dans une gamme de longueurs d'onde entre 400 nm et 490 nm, de préférence de lumière avec une longueur d'onde d'environ 405 nm et/ou d'une lumière avec une longueur d'onde d'environ 453 nm et/ou d'une lumière avec une longueur d'onde d'environ 470 nm.

7. Embout à insérer dans la bouche (201, 401, 601, 801, 1001, 1201, 1401, 1601) selon l'une des revendications 1 à 7, la source lumineuse (406) étant agencée pour une émission de lumière ou de rayonnement dans une gamme de longueurs d'ondes UV, par exemple de 222 nm, en plus de la plage de lumière bleue.

8. Embout à insérer dans la bouche (201, 401, 601, 801, 1001, 1201, 1401, 1601) selon l'une des revendications précédentes, le corps de cavité buccale (202, 402, 602, 1402, 1602) étant agencé pour une lumière émise de manière dirigée par la source lumineuse (406), cependant qu'il est prévu de préférence une région de pointe du corps de cavité buccale (202, 402, 602, 1402, 1602) en tant que zone d'émission de la lumière (210, 1580, 1580', 1780, 1780').

9. Embout à insérer dans la bouche (201, 401, 601, 801, 1001, 1201, 1401, 1601) selon l'une des revendications précédentes, l'embout à insérer dans la bouche (201, 401, 601, 801, 1001, 1201, 1401, 1601) ayant un corps en forme de bouton (611), un corps en forme de boîte (260, 860, 1660) et/ou un corps en forme de bloc (458, 1258, 1458), en particulier en tant que prise (264, 464), qui est situé au-delà de la surface d'appui (204, 404), cependant qu'en particulier le corps comme le corps en forme de bouton, de boîte ou de bloc (260, 458, 611), est équipé d'un branchement électrique (212) comme, par exemple un câble, une prise (305, 505) ou une fiche.

10. Embout à insérer dans la bouche (201, 401, 601, 801, 1001, 1201, 1401, 1601) selon l'une des revendications précédentes, le corps de cavité buccale (202, 402, 602, 1402, 1602) étant souple et/ou étanche au liquide, par exemple en raison d'une enveloppe en matière synthétique (282, 482) comme, par exemple en silicone, ou par exemple en raison d'une enveloppe en latex.

11. Embout à insérer dans la bouche (201, 401, 601, 801, 1001, 1201, 1401, 1601) selon l'une des revendications précédentes, l'embout à insérer dans la bouche (201, 401, 601, 801, 1001, 1201, 1401, 1601) ayant un corps de cavité buccale (202, 402, 602, 1402, 1602) composé symétriquement de deux demi-coques (213, 214), par exemple un corps transversal configuré ovale, qui peut former en particulier en raison d'une pression ou d'une dépression une surface de forme adaptable à la cavité buccale (20, 20') d'un utilisateur.

12. Embout à insérer dans la bouche (201, 401, 601, 801, 1001, 1201, 1401, 1601) selon l'une des revendications précédentes, le corps de cavité buccale (202, 402, 602, 1402, 1602) pouvant coulisser, en particulier pouvant être allongé et/ou déplacé, par rapport à un point de référence labial (269) de l'embout à insérer dans la bouche (201, 401, 601, 801, 1001, 1201, 1401, 1601), ou le corps de cavité buccale (202, 402, 602, 1402, 1602) pouvant être remplacé, en particulier en écartant ou en comprimant des ergots de retenue à clip (534", 959, 959', 1159, 1159'), par exemple par deux mouvements orientés de sens opposé, si bien que de préférence l'embout à insérer dans la bouche (201, 401, 601, 801, 1001, 1201, 1401, 1601) peut être modifié quant à sa longueur (203).

13. Embout à insérer dans la bouche (201, 401, 601, 801, 1001, 1201, 1401, 1601) selon l'une des revendications précédentes, cependant qu'il existe sur l'embout à insérer dans la bouche (201, 401, 601, 801, 1001, 1201, 1401, 1601) un bouton marche, par exemple un bouton de mise en marche avec verrouillage temporel (301, 501, 701, 1501, 1701), en particulier dans une zone latérale du corps.

14. Embout à insérer dans la bouche (201, 401, 601, 801, 1001, 1201, 1401, 1601) selon l'une des revendications précédentes, cependant que la lumière (1415, 1415^{I}, 1417, 1418, 1615, 1615^{I}, 1617, 1617^{I}, 1618, 1618^{I}), produite par plusieurs LED (571, 572, 573, 577, 578, 579, 961, 1361, 1367, 1368, 1561, 1565, 1761, 1765), possède, en plus de la plage de lumière bleue, une longueur d'onde dans la plage de lumière violette et/ou dans la plage de lumière UV.
